# EUROPEAN PATENT SPECIFICATION

(11) **EP 1 903 075 B1**
(45) Date of publication and mention of the grant of the patent: **27.01.2010**
(21) Application number: 07017859.5
(22) Date of filing: 12.09.2007
(51) Int. Cl.: C08L 51/00, G01N 33/533

(54) **Fluorescent polymer fine particle set, fluorescence detecting complex member set, use of fluorescent polymer fine particle set and fluorescence detecting method**
Feinteilchensatz von fluoreszierenden Polymeren, Satz von komplexen Elementen zur Fluoreszenz-Erkennung, Verwendung des Feinteilchensatzes aus fluoreszierenden Polymeren und Verfahren zur Fluoreszenz-Erkennung
Ensemble de particules fines de polymère fluorescent, ensemble de membres complexes de détection de la fluorescence, utilisation d'ensemble de particules fines de polymère fluorescent et procèdède détection de la fluorescence

(30) Priority: 19.09.2006 JP 2006252792
(43) Date of publication of application: 26.03.2008
(73) Proprietor: FUJIFILM Corporation, Minato-ku Tokyo (JP)
(72) Inventor: Makino, Naonori, c/o Fujifilm Corporation, Kaisei-machi, Ashigarakami-gun Kanangawa (JP)
(74) Representative: HOFFMANN EITLE

(56) References cited:
- EP-A- 0 195 623
- EP-A- 1 582 539
- EP-A- 1 783 168
- US-A- 4 259 313
- US-A- 5 284 752

## Description

### BACKGROUND OF THE INVENTION

### Field of the Invention

The present invention relates to a fluorescent polymer fine particle set, a fluorescence detecting complex member set, a fluorescent polymer fine particle composition, and a fluorescence detecting method.

In order to visualize or quantify a substance of a minute amount, various labeling substances have been developed. In fields requiring a particularly high sensitivity, radioisotopes are representative labeling substances, and tritium and radioactive iodine have been utilized as representative examples. However, as the radioactive substances involve various difficulties in disposal after use and in handling, methods alternative to the radioactive substances have been developed. Such methods include, for example, an enzyme labeling method (utilizing peroxidase, alkaline phosphatase, glucose oxidase or (β-D-galactosidase), and a fluorescent labeling method (utilizing fluorescein or rhodamine).

However, these methods involve a drawback of being deficient in the absolute sensitivity as a label.

In order to improve the precision and sensitivity of measurement, a time-resolved fluorescence measurement has been developed (cf. JP-A-61-128168) as an extension of the fluorescent labeling method. This method is based on irradiating a fluorescent substance of a long fluorescence extinction time, as represented by an europium chelate, with a pulsed excitation light, and measuring the fluorescence after a certain time namely after the termination of the direct excitation light and the extinction of fluorescence of short duration resulting from ambient substances, thereby measuring a fluorescence specific to europium.

It is also attempted, in order to further improve the sensitivity, to enclose such europium chelate or a dye in polystyrene particles, then to coat the surface of the polystyrene particles with an antigen or an antibody to prepare a reagent, and to visually detect the polystyrene particles immobilized by the antigen-antibody reaction (for example cf. JP-A-2000-345052).

However, such known method of preparing a labeling substance by enclosing a dye or a fluorescent substance in polystyrene particles, though being capable of attaining a certain sensitivity by simple operations, is insufficient in sensitivity, and a further improvement in the sensitivity has been desired.

Also because of the fact that the particle surface is constituted of hydrophobic polystyrene, the method has been utilized with various modifications such as, (1) after bonding a functional molecule such as an antigen or an antibody desired for coating, coating the unbonded surface with a protein or various biosubstance-like materials thereby masking the hydrophobicity of polystyrene, or (2) adding a surfactant in the liquid phase at the reaction thereby preventing mutual interaction of the polystyrene particles. Nevertheless, errors in judgment may result from a non-specific reaction.

On the other hand, in order to make an improvement on the non-specific reaction resulting from polystyrene particle, there have been developed a technology of polymerizing a hydrophilic macromonomer, having a reactive group at a terminal end of a polyethyleneoxy group and a radical polymerizable group at the other terminal end, and hydrophobic radical polymerizable monomer to form a core-shell type fine particle having a core formed by a water-insoluble polymer weight compound and a hydrophilic shell portion having a reactive group and incorporating a fluorescent dye in the core portion, and a composition for fluorescence analysis stably having a high fluorescence intensity thus attained (for example cf. W02002/097436 pamphlet).

Also proposed is a semiconductor nanoparticle fluorescent material, capable of detecting a target molecule by bonding a molecular probe to the surface of a bead prepared with a semiconductor nanoparticle such as of CdSe/CdS (core/shell) or CdSe/ZnS (core/shell) (for example Science, vol. 281, No. 25, p.2013-2016(1998) and Nature Biotechnology, vol. 19, p.631-635(2001)). Such semiconductor nanoparticle can provide a light emission of different wavelengths by assuming different crystal sizes. Also a simultaneous multiplex measurement is considered possible by encoding the labeled bead with a combination of the light emission wavelength and the light emission intensity.

However, the method utilizing such fine particles of core-shell configuration can suppress noises induced by the non-specific reactions but is unsatisfactory in the fluorescence intensity, and only one target substance of detection can be detected in a single operation. Also the labeled bead utilizing the semiconductor nanoparticle such as of CdSe/CdS or CdSe/ZnS (core/shell) involves a concern from the standpoints of safety and environmental effect, and is unsatisfactory in the sensitivity of detection.

Therefore, the present invention is to provide a fluorescent polymer fine particle set, a fluorescence detecting complex member set, the use of a fluorescent polymer fine particle set, and a fluorescence detecting method, capable of detecting plural target substances of detection simultaneously and with a high sensitivity.

### SUMMARY OF THE INVENTION

The present invention has been made in view of the above circumstances and provides a fluorescent polymer fine particle set, a fluorescence detecting complex member set, the use of a fluorescent polymer fine particle set, and a fluorescence detecting method.

A first aspect of the present invention provides:
[1] a fluorescent polymer fine particle set including at least a first fluorescent polymer fine particle containing a polymer fine particle having a core-shell configuration formed by a hydrophobic core and a hydrophilic shell, and a first fluorescent lanthanoid dye incorporated in the polymer fine particle and containing a lanthanoid cation; and a second fluorescent polymer fine particle containing the polymer fine particle, and a second fluorescent lanthanoid dye different from the first fluorescent lanthanoid dye.

A second aspect of the present invention provides:
[10] a fluorescence detecting complex member set including at least a first fluorescence detecting complex member, constituted of a first fluorescent polymer fine particle contained in the fluorescent polymer fine particle set as described in the first aspect [1] and a first binding material capable of binding the fluorescent polymer fine particle and a target substance of detection, and a second fluorescence detecting complex member, constituted of a second fluorescent polymer fine particle contained in the fluorescent polymer fine particle set and a second binding material different from the first binding material.

A third aspect of the present invention provides:
[12] The use of a fluorescent polymer fine particle set in a composition for a display apparatus; the fluorescent polymer fine particle set including at least a first fluorescent polymer fine particle containing a polymer fine particle having a core-shell configuration formed by a hydrophobic core and a hydrophilic shell, and a first fluorescent lanthanoid dye incorporated in the polymer fine particle and containing a lanthanoid cation; and a second fluorescent polymer fine particle containing the polymer fine particle, and a second fluorescent lanthanoid dye different from the first fluorescent lanthanoid dye.

A fourth aspect of the present invention provides:
[14] a fluorescence detecting method at least including a step of forming a first fluorescence detecting complex member from a first fluorescent polymer fine particle contained in the fluorescent polymer fine particle set as described in the first aspect [1] and with a first binding material capable of binding the fluorescent polymer fine particle and a target substance of detection, a step of forming a second fluorescence detecting complex member, from a second fluorescent polymer fine particle contained in the fluorescent polymer fine particle set as described in the first aspect [1], and a second binding material different from the first binding material, a step of bringing the first and second fluorescence detecting complex members into contact with a specimen containing a target substance for detection, a first detection step of detecting the first fluorescent lanthanoid dye contained in the first fluorescence detecting complex member, and a second detection step of detecting the second fluorescent lanthanoid dye contained in the second fluorescence detecting complex member.

A fifth aspect of the present invention provides:
[15] a fluorescence detecting method including at least a step of bringing the fluorescence detecting complex members contained in the fluorescence detecting complex member set as described in the second aspect [10] into contact with a specimen containing a target substance for detection, a first detection step of detecting the first fluorescent lanthanoid dye contained in the first fluorescence detecting complex member, and a second detection step of detecting the second fluorescent lanthanoid dye contained in the second fluorescence detecting complex member.

### DETAILED DESCRIPTION OF THE INVENTION

The present invention enables to provide a fluorescent polymer fine particle set, a fluorescence detecting complex member set, the use of a fluorescent polymer fine particle set, and a fluorescence detecting method, capable of simultaneously detecting plural target substances for detection with a high sensitivity.

### [Fluorescent polymer fine particle set]

The fluorescent polymer fine particle set of the present invention is characterized in including at least a first fluorescent polymer fine particle containing a polymer fine particle having a core-shell configuration formed by a hydrophobic core and a hydrophilic shell, and a first fluorescent lanthanoid dye incorporated in the polymer fine particle and containing a lanthanoid cation, and a second fluorescent polymer fine particle containing the polymer fine particle, and a second fluorescent lanthanoid dye different from the first fluorescent lanthanoid dye.

The fluorescent lanthanoid dyes, being respectively contained in the first fluorescent polymer fine particle and in the second fluorescent polymer fine particle and being respectively different, allow to separately detect the respective fluorescent polymer fine particles corresponding to the target substances of detection in a single operation and with high sensitivities.

In the present invention, the two fluorescent lanthanoid dyes being different means that the fluorescent lanthanoid dyes have different structures. The lanthanoid dyes being different in the structure means, for example, a difference in the type of the lanthanoid cation and/or a difference in an organic ligand coordinated with the lanthanoid cation. As will be described later, a control on the structure of the lanthanoid dye can arbitrarily control an excitation wavelength and/or a light emission wavelength.

In the present invention, examples of the mode where the compared fluorescent lanthanoid dyes are different include a mode where the dyes are different in the excitation wavelength but same in the light emission wavelength, a mode where the dyes are same in the excitation wavelength but different in the light emission wavelength, and a mode where the dyes are different in the excitation wavelength and the light emission wavelength.

In one embodiment of the present invention,the dyes are same in the excitation wavelength but different in the light emission wavelength, or the dyes are different in the excitation wavelength and the light emission wavelength, and more preferred is an embodiment where the dyes are same in the excitation wavelength but different in the light emission wavelength.

Also the fluorescent polymer fine particle set of the present invention may further include at least one third fluorescent polymer fine particle containing the polymer fine particle and a third lanthanoid dye different from the first and second fluorescent lanthanoid dyes. It is thus rendered possible to separately detect the fluorescent polymer fine particles corresponding to the target substances of detection of arbitrary types in a single operation and with high sensitivities.

In the invention, it is preferable that at least one of organic ligands contained in the first fluorescent lanthanoid dye and at least one of organic ligands contained in the second fluorescent lanthanoid dye are different from each other. The respectively different organic ligands in the plural fluorescent lanthanoid dyes allow to obtain respectively different excitation wavelengths in the plural fluorescent lanthanoid dyes. In this manner, the plural fluorescent polymer fine particles containing different fluorescent lanthanoid dyes can be separately detected.

In the invention, the first fluorescent lanthanoid dye and the second fluorescent lanthanoid dye preferably have a difference in the excitation wavelength of 10 nm or larger, more preferably 50 nm or larger. A difference in the excitation wavelength of 10 nm or larger enables to detect, separately and more easily, the plural fluorescent polymer fine particles containing respectively different fluorescent lanthanoid dyes.

In the invention, it is preferable that the lanthanoid cation contained in the first fluorescent lanthanoid dye and the lanthanoid cation contained in the second fluorescent lanthanoid dye are different from each other. The respectively different lanthanoid cations in the plural fluorescent lanthanoid dyes allow to obtain respectively different light emission wavelengths where the light emission intensities of the plural fluorescent lanthanoid dyes become maximum (peak light emission wavelengths). In this manner, the plural fluorescent polymer fine particles containing different fluorescent lanthanoid dyes can be separately detected.

In the invention, it is also preferable that the first fluorescent lanthanoid dye and the second fluorescent lanthanoid dye have a difference in the peak light emission wavelengths of 10 nm or larger. A difference in the peak light emission wavelengths of 10 nm or larger enables to detect, separately and more easily, the plural fluorescent polymer fine particles containing respectively different fluorescent lanthanoid dyes.

In the fluorescent polymer fine particle set of the present invention, the micromonomer constituting the hydrophilic shell of the polymer fine particle and the monomer constituting the hydrophobic core are not particularly restricted, but the polymer particle is preferably such that the hydrophilic shell is formed at least by a hydrophilic (meth)acrylic macromonomer or a hydrophilic vinyl macromonomer, and the hydrophobic core is formed at least by a hydrophobic (meth)acrylic monomer or a hydrophobic vinyl monomer.

The hydrophilic shell may further contain a hydrophilic monomer or a hydrophilic macromonomer other than the hydrophilic (meth)acrylic macromonomer or the hydrophilic vinyl macromonomer, and the hydrophobic core may further contain a hydrophobic monomer other than the hydrophobic (meth)acrylic monomer or the hydrophobic vinyl monomer.

In the invention, the above-described construction of the polymer fine particle suppresses a non-specific adsorption, thereby improving the stability in time of the fluorescent polymer fine particle.

Also in the invention, the hydrophilic (meth)acrylic macromonomer or the hydrophilic vinyl macromonomer preferably contains a reactive functional group. In this manner, the hydrophilic shell of the polymer particle includes a reactive functional group, whereby the combination substance capable of binding the polymer particle and the target substance of detection can be reacted at a high efficiency to further improve the detection sensitivity.

In the following, an example of the construction of the present invention is shown.

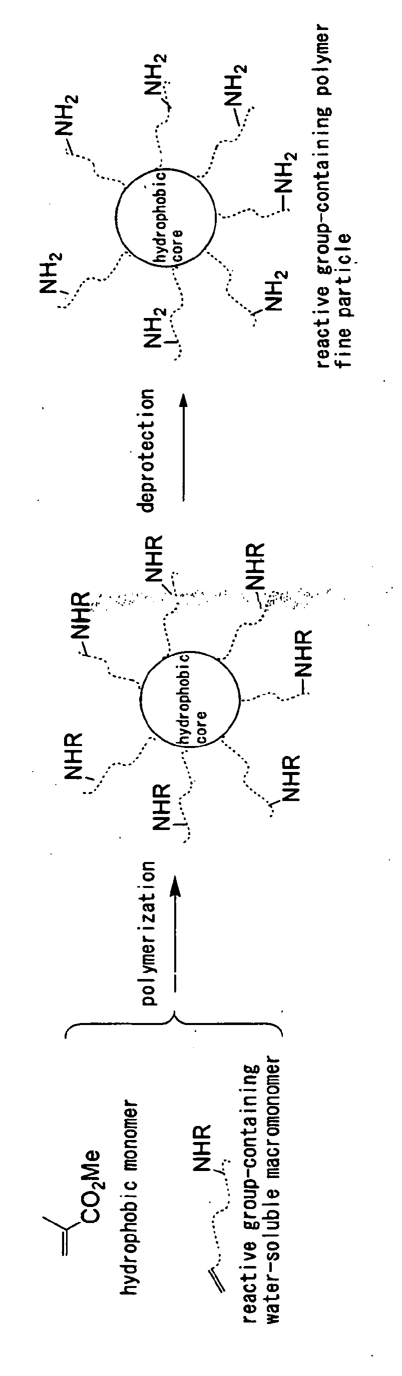

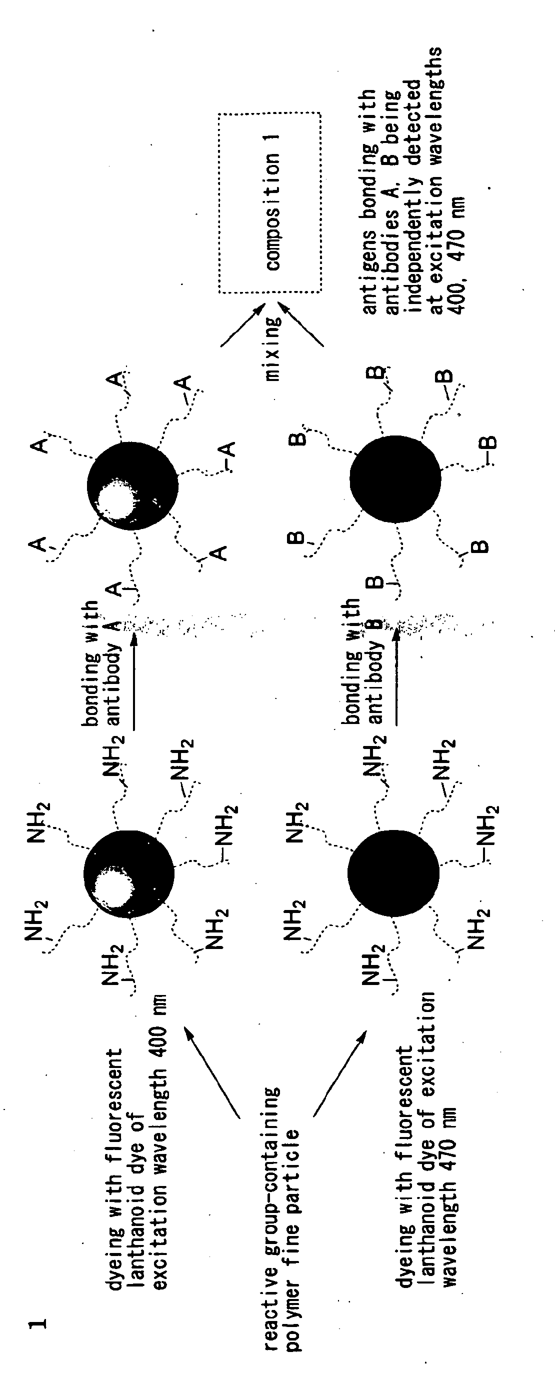

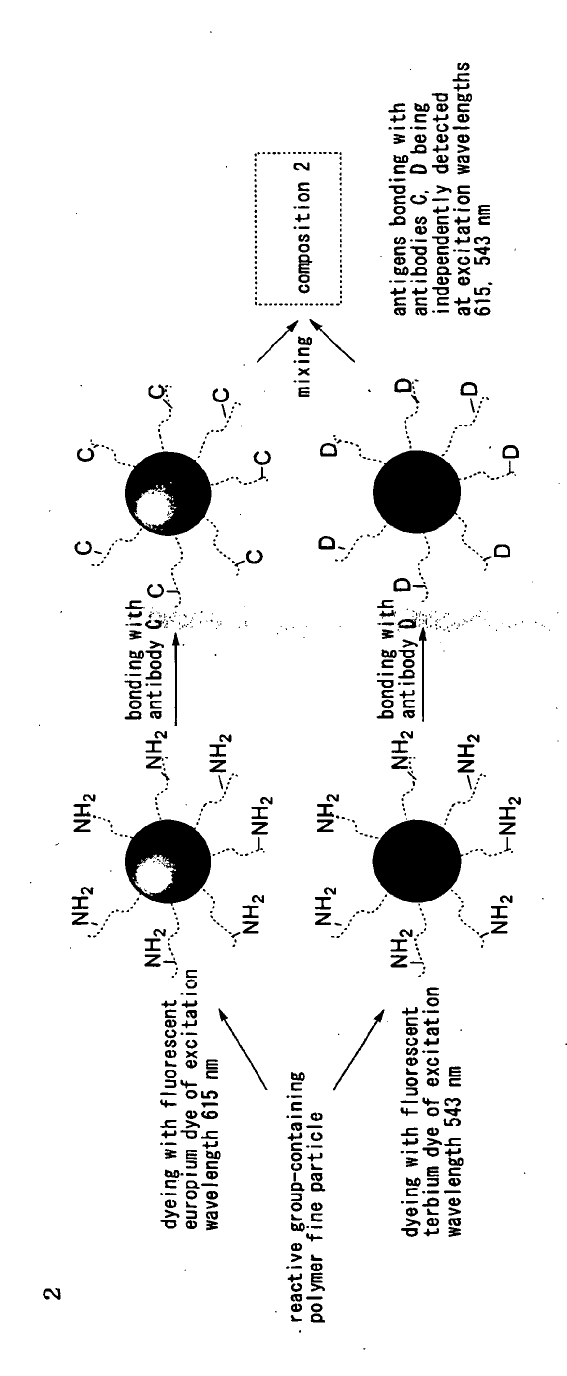

In the following, the present invention will be explained in further details.

### <Fluorescent polymer fine particle>

### (Hydrophilic macromonomer)

The hydrophilic (meth)acrylic macromonomer and the hydrophilic vinyl macromonomer to be employed in the present invention include a polymerizable double bond at a terminal end of a polymer main chain. As will be described later, the hydrophilic macromonomer constitutes a hydrophilic shell, by reacting, in an aqueous solvent, with radical species that is generated by a reaction between a radical-polymerizable monomer and a polymerization initiator, and forms a core-shell type resin fine particle, in which a hydrophobic polymer, constituted of a hydrophobic radical-polymerizable monomer bonded to a terminal end of the hydrophilic macromonomer forms a hydrophobic core.

Also the hydrophilic macromonomer in the invention preferably has a reactive functional group capable of linking with the combination substance (for example a physiologically active substance (such as antibody, enzyme or nucleic acid)).

The reactive functional group may be introduced in the hydrophilic macromonomer at the formation of the hydrophilic polymer, and/or may be introduced in the hydrophilic polymer after bonding to the surface of the core portion to be explained later. It is preferably introduced as a reactive functional group at the formation of the hydrophilic macromonomer, since such mode enables to regulate the introducing rate of the reactive functional group.

Such reactive functional group is not particularly restricted as long as it is stable in water (or an aqueous solvent) and is capable of reacting with a combination substance such as a physiologically active substance (such as an enzyme, DNA or the like) in a case where the fluorescent polymer particle of the invention is used as a labeled substance. Preferable examples of the functional group to be provided on the surface of the polymer fine particle include an aldehyde group, a carboxyl group, a mercapto group, an acid halide group such as an acid chloride group, an acid anhydride group, an ester group, an amide group, a maleimide group, a vinylsulfone group, a methanesulfonyl group, a thiol group, a hydroxyl group, and an amino group. More preferable examples among these include an aldehyde group, a carboxyl group, an acid chloride group, an acid anhydride group, a maleimide group, a thiol group, and an amino group. Most preferable examples among these include an aldehyde group, a maleimide group, a thiol group, and an amino group. Since an ester group can be easily converted into a carboxyl group by hydrolysis, the polymer fine particle having an ester group on the surface is a useful intermediate.

An amount of the functional groups for bonding the combination substance, represented in equivalents of the functional groups per unit weight of the polymer fine particles of the invention, is normally from 0.001 to 0.5 meq/g, preferably from 0.005 to 0.2 meq/g, more preferably from 0.01 to 0.1 meq/g, further preferably from 0.02 to 0.07 meq/g, and most preferably from 0.03 to 0.05 meq/g.

The hydrophilic macromonomer is preferably a macromonomer represented by the following formula (I) or (II), in consideration of the particle forming property and the reactivity with the combination substance to be described later.

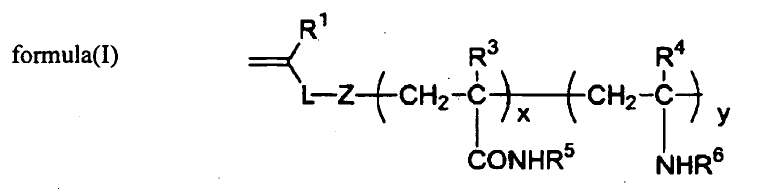

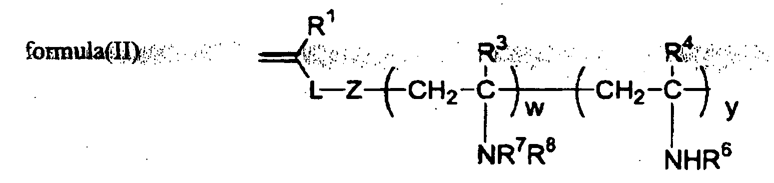

In the formula (I), R¹, R³ and R⁴ each independently represent a hydrogen atom, an alkyl group having 1 to 6 carbon atoms, or a halogen atom. Specific examples of alkyl group include a methyl group, an ethyl group, a propyl group, a butyl group, a pentyl group and a hexyl group, and may be linear or cyclic. Also the alkyl group may have a substituent, and examples thereof include an aryl group, a hydroxyl group, an amino group, a heterocyclic group, an alkylthio group, an arylthio group, an alkoxy group, an alkoxycarbonyl group, an aryloxy group, an amide group, an ureido group, a halogen atom (fluorine, chlorine, bromine or iodine), and a cyano group. In the cited examples, the substituent preferably contains, excluding hydrogen atom, 1 to 50 atoms, more preferably 1 to 30 atoms and most preferably 1 to 20 atoms. In the case that aryl group has a substituent, preferable examples of the substituent include an alkyl group, an alkenyl group, an alkinyl group, an aryl group, a heterocyclic group, a silyl group, an alkoxy group, an amino group, an alkylamino group, a dialkylamino group, an acylamino group, an alkyl- or aryl-sulfonylamino group, an acyl group, an alkyl- or aryl-sulfonyl group, a formyl group, an alkoxycarbonyl group, a carbamoyl group, a sulfamoyl group, a halogen atom, a cyano group, a sulfo group and a carboxyl group. R¹, R³ and R⁴ each is preferably a hydrogen atom or a methyl group, in view of particle formation.

R⁵ represents a hydrogen atom or an alkyl group having 1 to 4 carbon atoms. Specific examples of the alkyl group include a methyl group, an ethyl group, a propyl group, an isopropyl group and a butyl group. Among these, R⁵ is preferably a hydrogen atom or a methyl group in consideration of hydrophilicity of the macromonomer, particularly preferably a hydrogen atom.

R⁶ represents a hydrogen atom, a formyl group or an acetyl group.

R⁷ and R⁸ each independently represent a hydrogen atom or an alkyl group having 1 to 6 carbon atoms, and also may be bonded with each other to form a nitrogen-containing ring. Examples of the alkyl group include a methyl group, an ethyl group, and a propyl group. In the case that a nitrogen-containing ring is formed by bonding with each other, examples of the nitrogen-containing ring include a pyrrolidine ring, a piperadine ring, a morpholine ring, a pyrrolidone ring, and a pyrrole ring. In view of the hydrophilicity and particle forming property of macromonomer, R⁷ and R⁸ are preferably such that R⁷ is a methyl group and R⁸ is a hydrogen atom, or that R⁷ and R⁸ constitute a pyrrolidone ring.

L represents a divalent atomic group, of which examples include a single bond, an alkylene group having 1 to 4 carbon atoms (such as a methylene group, an ethylene group, or a propylene group), an alkyleneoxy group having 1 to 6 carbon atoms (such as an ethyleneoxy group or a propyleneoxy group), -COO-, -OCO-, - (CH₂)ₜCOO-, -(CH₂)ₖOCO-, -O-, -SO₂-, -CONHCOO-, -CONHCONH-, -CON(R⁹)-, -SO₂N(R⁹)- (wherein R⁹ represents a hydrogen atom or a hydrocarbon atom such as an alkyl group, having 1 to 22 carbon atoms; t represents an integer of from 1 to 3; and k represents an integer of from 1 to 4), and an atomic group represented by the following formula (IA): 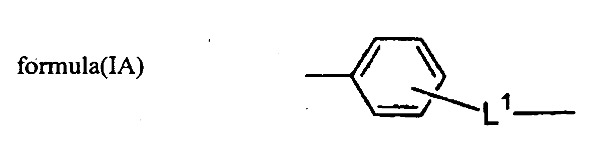 (wherein L¹ represents a single bond, a methylene group, -O-, -OCO- or -COO-), or a divalent atomic group formed by a combination of these. In the case that L is an alkylene group, an alkyleneoxy group or an atomic group represented by the formula (IA), it may have a substituent, and examples of such substituent include an alkyl group having 1 to 6 carbon atoms, and substituents same as those when R¹, R³ or R⁴ above is an alkyl group.

Also in the linking group -CON(R⁹)- or -SO₂N(R⁹)- represented by L, R⁹ preferably represents a hydrogen atom or an alkyl group such as a methyl group, an ethyl group, a propyl group, a butyl group, a pentyl group, a hexyl group, a heptyl group, an octyl group, a decyl group or a dodecyl group.

Z represents a bond directly linking to a terminal end of the polymer main chain, or a bonding group via an arbitrary linking group.

The linking group is formed by an arbitrary combination of atomic groups constituting a carbon atom-carbon atom bond (a single bond or a double bond), a carbon atom-hetero atom bond (hetero atom being for example an oxygen atom, a sulfur atom, a nitrogen atom or a silicon atom), and a hetero atom-hetero atom bond, including examples shown below:

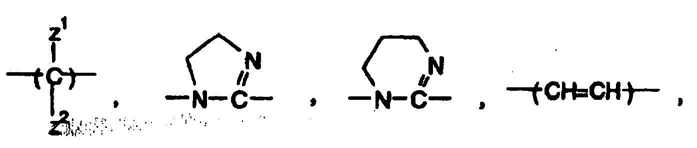

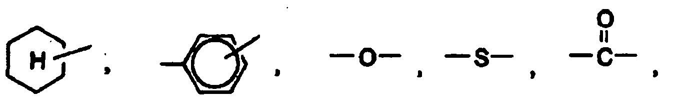

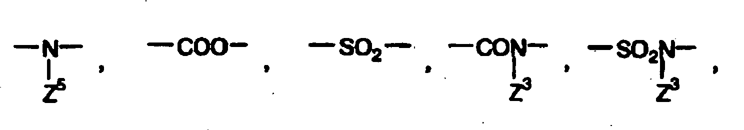

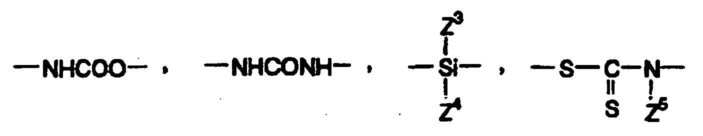

Z¹ and Z² each represent a hydrogen atom, a halogen atom (such as a fluorine atom, a chlorine atom, or a bromine atom), a cyano group, a hydroxyl group, or an alkyl group (such as a methyl group, an ethyl group or a propyl group). Z³ and Z⁴ each represent a hydrogen atom, a hydrocarbon group having 1 to 8 carbon atoms (such as a methyl group, an ethyl group, a propyl group, a butyl group, a pentyl group, a hexyl group, a benzyl group, a phenethyl group, a phenyl group or a tolyl group), or _OZ⁵, wherein Z⁵ have the same meaning as the hydrocarbon group in Z³.

Each of x and y represent a number of repeat of 1 or larger.

In case of assuming that a sum of x and y is 100, x is preferably within a range of from 10 to 99 in consideration of the hydrophilicity of macromonomer, more preferably from 20 to 99 and particularly preferably from 60 to 99. In such case, y is preferably within a range of from 1 to 60 in consideration of the introduced amount of the combination substance, more preferably from 2 to 50 and particularly preferably from 5 to 40.

W represents a number of repeat equal to or larger than 0.

The hydrophilic acrylamide type macromonomer of the present invention can be easily produced either by a method of reacting various reagents containing various double bond groups, with a terminal end of a polymer which is obtained by a known radical polymerization (for example iniferter process), an anionic polymerization or a cationic polymerization, utilizing a known polymerizable monomer as represented by any one of the following formulae (IV), (V) and (VI), or reacting a reagent containing a specified reactive group (such as -OH, -COOH, - SO₃H -NH₂, -SH, -PO₃H₂ -NCO, -NCS, 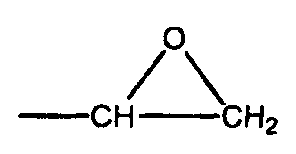 -COCl or -SO₂Cl) with a terminal end of such polymer, and then introducing a polymerizable double bond group by a polymer reaction (method by ionic polymerization), or by a method of executing a radical polymerization with a polymerization initiator and/or a chain transfer agent, containing the aforementioned specified reactive group within the molecule, and then executing a polymer reaction utilizing the specified reactive group bonded to a terminal end of the polymer main chain thereby introducing a polymerizable double bond group.

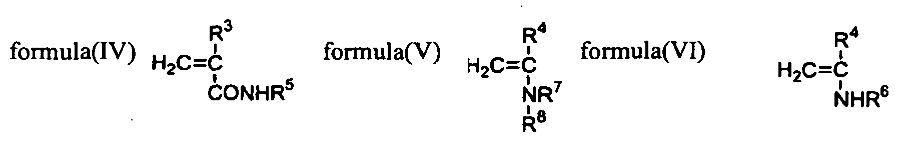

In the formulae (IV), (V) and (VI), R³, R⁴, R⁵, R⁶, R⁷ and R⁸ have the same meanings as R³, R⁴, R⁵, R⁶, R⁷ and R⁸ above.

More specifically, the polymerizable double bond can be introduced according to the methods described in general reports such as Takayuki Otsu, Kobunshi, 33(No. 3), 222(1984), P. Dreyfuss & R. P. Quirk, Encycl. Polym. Sci. Eng., 7,551(1987), Yoshiki Nakajo & Yuya Yamashita, Senryo to Yakuhin, 30, 232(1985), Akira Ueda & Susumu Nagai, Kagaku to Kogyo, 60, 57(1986), P. F. Rempp & E. Franta, Advances in Polymer Science, 58, 1(1984), Koichi Itoh, Kobunshi Kako, 35, 262(1986), and V. Percec, Applied Polymer Science, 285, 97(1984), and references cited therein.

More specific examples include a method of synthesizing a polymer in which a specified reactive group is bonded to a terminal end of the polymer main chain by (a) a method of polymerizing a mixture of at least a monomer corresponding to the repeating unit represented by any one of the formulae (IV), (V) and (VI) and a chain transfer agent containing the aforementioned specified reactive group within the molecule, utilizing a polymerization initiator (such as an azobis compound or a peroxide), (b) a method of polymerization without utilizing the above-mentioned chain trasnfer agent but utilizing a polymerization initiator containing the aforementioned specified reactive group within the molecule, or (c) a method of utilizing a chain transfer agent and a polymerization initiator both containing the aforementioned specified reactive group within the molecule, and then introducing a polymerizable double bond group by a polymer reaction utilizing such specified reactive group.

Examples of the usable chain transfer agent include mercapto compounds containing a specified reactive group or a substituent which can be derived to a specified reactive group [such as thioglycolic acid, thiomalic acid, thiosalicylic acid, 2-mercaptopropionic acid, 3-mercaptopropionic acid, 3-mercaptobutyric acid, N-(2-mercaptopropionyl)glycine, 2-mercaptonicotic acid, 3-[N-(2-mercaptoethyl)carbamoyl]propionic acid, 3-[N-(2-mercaptoethyl)amino]propionic acid, N-(3-mercaptopropionyl)alanine, 2-mercaptoethanesulfonic acid, 3-mercaptopropanesulfonic acid, 4-mercaptobutanesulfonic acid, 2-mercaptoethanol, 1-mercapto-2-propanol, 3-mercapto-2-butanol, mercaptophenol, 2-mercaptoethylamine, 2-mercaptoimidazole, or 2-mercapto-3-pyridinol], and iodoalkyl compounds containing a specified reactive group or a substituent which can be derived to a specified reactive group [such as iodoacetic acid, iodopropionic acid, 2-iodoethanol, 2-iodoethanesulfonic acid, or 3-iodopropanesulfonic acid], and the mercapto compounds are preferable.

Also examples of the polymerization initiator containing a specified reactive group or a substituent which can be derived to a specified reactive group include azobis compounds [such as 4,4'-azobis(4-cyanovaleric acid), 4,4'-azobis(4-cyanovaleryl chloride), 2,2'-azobis(2-cyanopropanol), 2,2'-azobis(2-cyanopentaol), 2,2'-azobis[2-(5-hydroxy-3,4,5,6-tetrahydropyrimidin-2-yl)propane], 2,2'-azobis[2-methyl-N-[1,1-bis(hydroxymethyl)-2-hydroxyethyl]propionamide], 2,2'-azobis[2-methyl-N-[1,1-bis(hydroxymethyl)-ethyl]propionamide], 2,2'-azobis[2-methyl-N-(2-hydroxyethyl)-propionamide], and 2,2'-azobis(2-amidinopropane)], and thiocarbamate compounds [such as benzyl-N-methyl-N-hydroxyethyl dithiocarbamate, 2-carboxyethyl-N,N-diethyl dithiocarbamate, and 3-hydroxypropyl-N,N-dimethl dithiocarbamate].

Such chain transfer agent or polymerization initiator is employed in an amount of from 0.01 to 10 parts by mass, preferably from 0.05 to 5 parts by mass in view of particle formation, with respect to 100 parts by mass of all the monomers. The number-average molecular weight of the hydrophilic macromonomer is not particularly restricted, but, in view of particle formation, preferably from 500 to 200,000, more preferably from 1,000 to 100,000 and further preferably from 2,000 to 50,000.

In synthesizing the hydrophilic macromonomer, for the purpose of regulating the hydrophilicity and the solubility in solvents, a known polymerizable monomer may be copolymerized in addition to the monomers represented by any one of the formulae (IV), (V) and (VI).

Examples of the known polymerizable monomer include styrenic monomers such as styrene, methylstyrene, chloromethylstyrene, 4-methoxystyrene, and 4-acetoxystyrene; (meth)acrylate esters such as methyl (meth)acrylate, ethyl (meth)acrylate, hydroxyethyl (meth)acrylate, propyl (meth)acrylate, butyl (meth)acrylate, hexyl (meth)acrylate, octyl (meth)acrylate, 2-ethylhexyl (meth)acrylate, cyclohexyl (meth)acrylate, dodecyl (meth)acrylate, stearyl (meth)acrylate, phenyl (meth)acrylate, benzyl (meth)acrylate, and 2-hydroxyethyl (meth)acrylate, and vinyl monomers such as vinyl acetate, and vinylimidazole. Such polymerizable monomer may be copolymerized preferably in an amount of from 0.1 to 30 mol%, and more preferably from 1 to 10 mol%, with respect to all the polymerizable monomers.

Specific examples of the hydrophilic macromonomers are shown below.

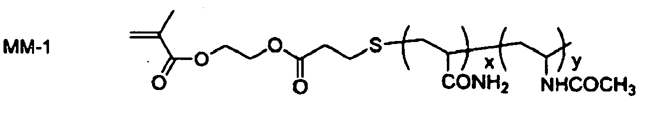

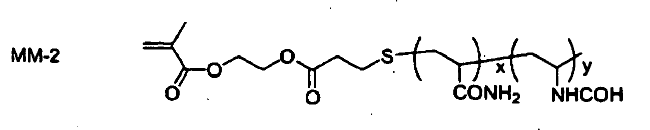

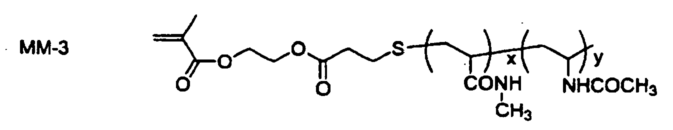

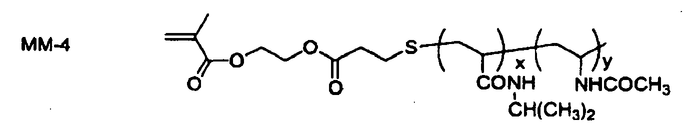

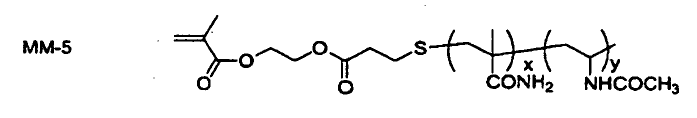

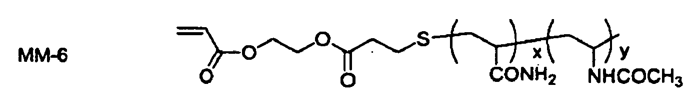

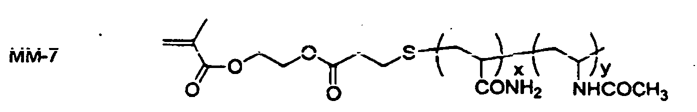

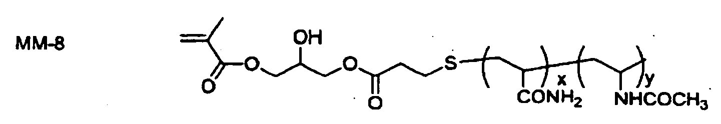

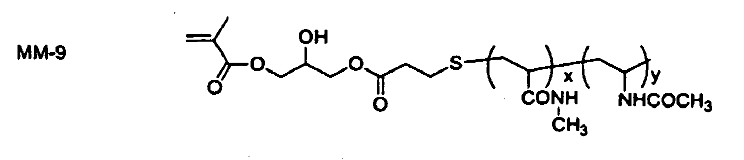

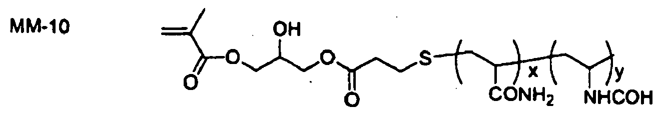

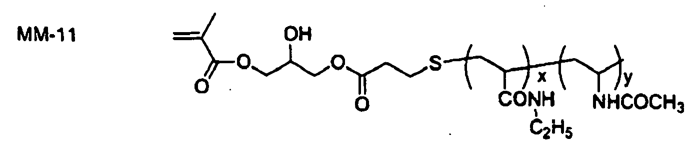

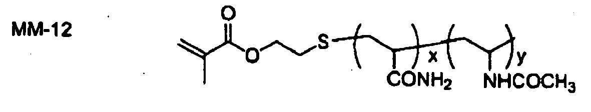

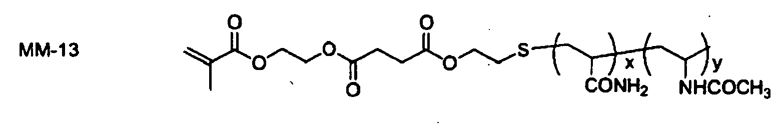

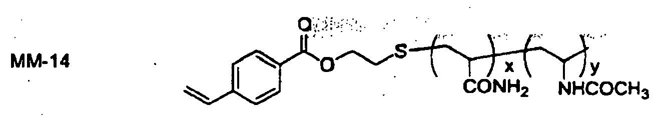

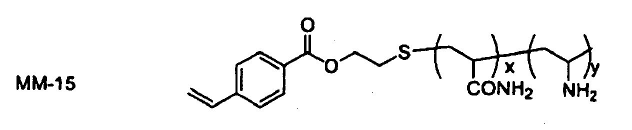

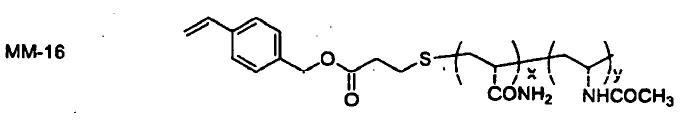

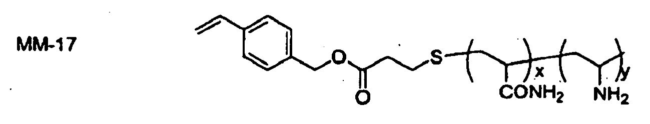

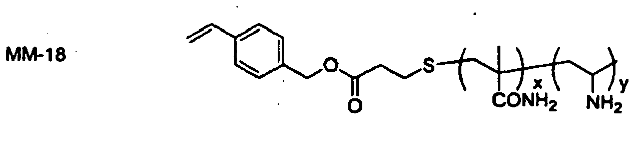

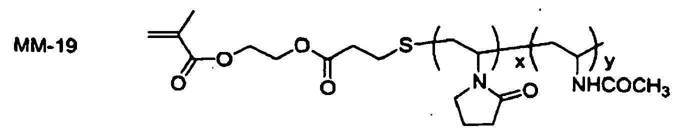

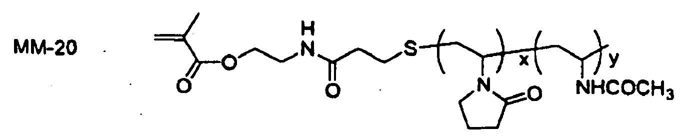

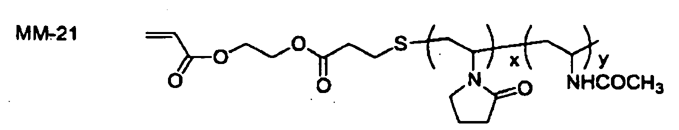

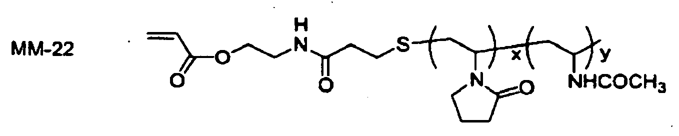

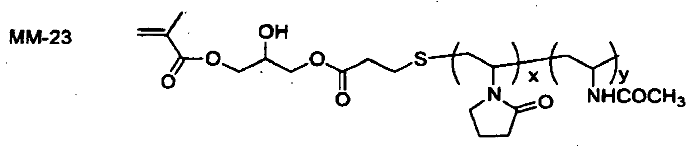

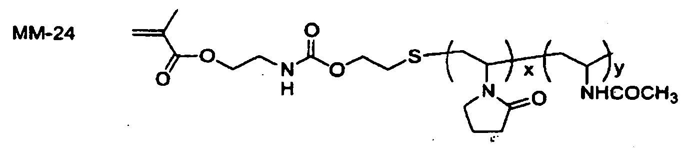

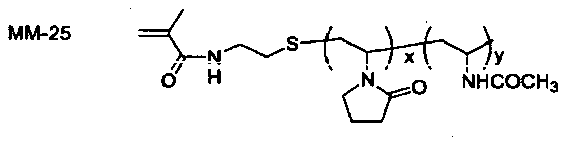

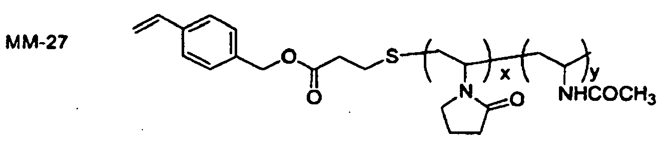

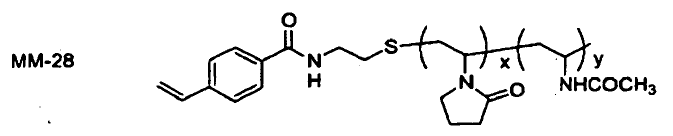

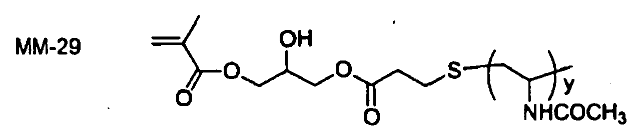

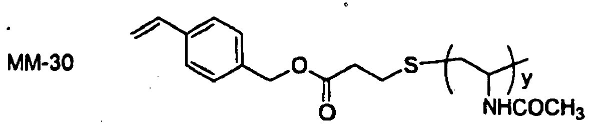

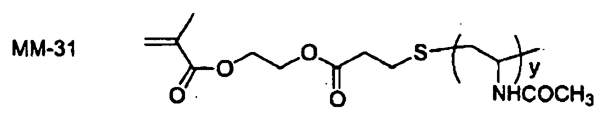

Also in the invention, for the purpose of assisting the particle formation, a hydrophilic macromonomer other than the aforementioned hydrophilic macromonomer may be used in combination. A specific example thereof may be a polyalkylenoxy group-containing hydrophilic macromonomer, for which a commercial macromonomer may be used. Specific examples of the commercial product include Blemmer (trade name, hereinafter same) PE-90, Blemmer PE-200, Blemmer PE-350, Blemmer AE-90, Blemmer AE-200, Blemmer AE-350, Blemmer 70PEP-350B, Blemmer AEP, Blemmer 55PET-800, Blemmer PME-100, Blemmer PME-200, Blemmer PME-400, Blemmer PME-1000, Blemmer PME-4000 and Blemmer AME-400 manufactured by NOF Corporation, and methoxypolyethylene glycol acrylate AM-90G, methoxypolyethylene glycol acrylate AM-230G, methoxypolyethylene glycol methacrylate M-90G and methoxypolyethylene glycol methacrylate M230 manufactured by Shin-Nakamura Chemical Industries Co. (Hydrophobic mulcal-polymerizable monomer)

As the hydrophobic radical-polymerizable monomer, a known radical-polymerizable monomer may be used.

Examples of the known radical-polymerizable monomer include styrenic monomers such as styrene, methylstyrene, chloromethylstyrene, 4-methoxystyrene and 4-acetoxystyrene; (meth)acrylate esters such as methyl (meth)acrylate, ethyl (meth)acrylate, propyl (meth)acrylate, butyl (meth)acrylate, hexyl (meth)acrylate, octyl (meth)acrylate, 2-ethylhexyl (meth)acrylate, cyclohexyl (meth)acrylate, dodecyl (meth)acrylate, stearyl (meth)acrylate, phenyl (meth)acrylate, benzyl (meth)acrylate, and 2-hydroxyethyl (meth)acrylate; and vinyl monomers such as vinyl acetate, vinyl chloride, vinylimidazole and vinylpyridine. Such monomers may be polymerized in one kind or copolymerized in two or more kinds.

In the present invention, in view of the stability of the fluorescent polymer fine particle, at least one of the hydrophobic radical-polymerizable monomers is preferably a monomer having a polar group. Examples of the polar group include an alkoxy group, an ester group, an acetoacetyl group and a heterocyclic group.

In the hydrophobic core of the polymer fine particle, an interaction of the polar group and the fluorescent lanthanoid dye enables the fluorescent lanthanoid dye to be present more stably on the hydrophobic core. Thus, even in a state where plural fluorescent polymer fine particles having different fluorescent lanthanoid dyes are present in a mixture, an exchange of the fluorescent lanthanoid dyes between the different fluorescent polymer fine particles can be more efficiently suppressed. Thus, the detection sensitivity and the detection specificity can be more improved at the detection of the fluorescent lanthanoid dyes.

For the purpose of improving the strength of the particle, a crosslinked structure may be introduced into the polymer of the particle. For this purpose, a divalent radical-polymerizable monomer such as divinylbenzene or ethylene glycol dimethacrylate may be utilized. The divalent radical-polymerizable monomer may be employed within a range of from 1 to 20 mol% with respect to all the radical-polymerizable monomers constituting the particle, more preferably from 2 to 10 mol%.

### (Polymer fine particle)

The polymer fine particles are not particularly restricted in the particle size, but a volume-average particle size is normally selected within a range of from 0.01 to 20 µm. Particularly in case of use as a fluorescence detecting complex member, the volume-average particle size is preferably within a range of from 0.01 to 2 µm. A volume-average particle size of 1 µm or less allows to suppress problems such as a sedimentation property, and a volume-average particle size of 0.01 µm or more ensures a satisfactory operability. For these reasons, the polymer fine particles of the invention more preferably have a volume-average particle size of from 0.05 to 1 µm, further preferably from 0.05 to 0.5 µm, and most preferably from 0.05 to 0.3 µm. The volume-average particle size can be measured by an ordinary measuring method, and can be easily measured, for example, with a particle size distribution measuring apparatus (Coulter N4Plus, manufactured by Beckman-Coulter Inc.), or Nanotrack particle size analyzer UPA-EX (manufactured by Nikkiso Co.).

A shape of the core portion is not particularly limited, but is generally approximately spherical or approximately ellipsoidal. Also a dimension of the core portion is not particularly limited, and may be suitably changed according to the purpose, but the core portion of approximately spherical shape generally has a diameter of about from 5 to 200 nm.

A ratio of the diameter of the core portion and the thickness of the shell portion may be changed suitably according to the purpose. For example, the diameter of the core portion may be selected as from 5 to 200 nm, and the thickness of the shell portion may be selected as from 5 to 500 nm.

### (Producing method of polymer fine particle)

The polymer fine particle of the present invention can be produced, in the presence of the hydrophilic (meth)acrylic macromonomer or the hydrophilic vinyl macromonomer described above and the radical generator, by dispersing the hydrophobic radical-polymerizable monomer such as the styrene derivative or the (meth)acrylic acid derivative in an aqueous solvent, and executing a radical polymerization.

In the polymerization, there may be adopted a method of mixing and dissolving the entire amounts of the radical-polymerizable monomer and the hydrophilic macromonomer in an aqueous solvent, namely water or a water-containing organic solvent, and adding a radical generator (polymerization initiator), but, in order to control the reaction temperature and the particle size, preferred also is a method of gradually adding the radical-polymerizable monomer and the polymerization initiator into water or a water-containing organic solvent, in which the hydrophilic macromonomer is dissolved in advance.

An amount of the hydrophilic macromonomer may be suitably regulated according to the desired particle size, the ratio of the core portion and the shell portion, and the type of the radical-polymerizable monomer. In consideration of the particle forming property, it may be employed within a range of from 1 to 300 mass% with respect to the total weight of the radical-polymerizable monomer, more preferably from 20 to 200 mass% in consideration of the particle size of the fine particles obtained.

Preferable examples of the water-containing organic solvent include mixtures of water and methanol, ethanol, acetone, N,N-dimethylformamide, N,N-dimethylacetamide, tetrahydrofuran or dimethylsulfoxide.

Common radical generator are water-soluble radical initiator, for example, persulfate salts such as sodium persulfate, potassium persulfate, lithium persulfate and ammonium persulfate, or those soluble in the radical-polymerizable monomer or the water-containing organic solvent, for example an azo compound such as 2,2'-azobisisobutyronitrile (AIBN) or 2,2'-azobis(2,4-dimethylvaleronitrile) and peroxides such as benzoyl peroxide. Also different polymerization initiators may be used in combination. Also, the polymerization initiator is preferably employed within a range of from 0.01 to 5 mass% with respect to the total weight of the radical-polymerizable monomer, and more preferably from 0.1 to 3 mass%.

A reaction temperature is suitably selected according to the decomposition temperature of the initiator to be employed, and is preferably from 40 to 100°C and more preferably from 60 to 80°C.

In the fluorescent polymer fine particle of the invention, it is also possible to use an arbitrary additive, capable of coordination with the lanthanoid cation thereby suppressing a loss in the fluorescence intensity for example by a hydration, for example an organic phosphorus compound such as trioctylphosphine oxide or tributylphosphine oxide, as long as the intent of the invention is not significantly affected, and such additive is advantageously added in advance to the monomer solution at the polymerization.

In the following, the fluorescent polymer fine particle of the invention will be described. The fluorescent polymer fine particle includes the polymer particle above and the fluorescent lanthanoid dye incorporated therein and containing a lanthanoid cation.

### (Fluorescent lanthanoid dye)

The fluorescent lanthanoid dye to be employed in the present invention is a fluorescent dye, including a lanthanoid cation and at least an organic ligand. Such fluorescent dye can be excited with a visible light, and exhibits a sensitizing effect of the ligand (a phenomenon of light emission from the lanthanoid cation by the energy of light exciting the ligand).

Such ligand is preferably a ligand including a nitrogen-containing heterocycle, having a high fluorescence intensity and a long fluorescence lifetime as represented by the formula (L-1).

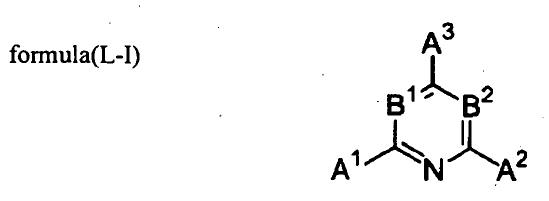

In the formula (L-1), A¹, A² and A³ each represent an atomic group represented by any one of the following formulae (L-II) to (L-V), a hydroxyl group, an alkoxy group, an aryloxy group, an alkylamino group, a dialkylamino group, an arylamino group or a diarylamino group, and may be same with one another. In the case that each represents an alkoxy group, an aryloxy group, an alkylamino group, a dialkylamino group, an arylamino group or a diarylamino group, it preferably has 1 to 12 carbon atoms in consideration of ease of inclusion in the polymer fine particle and solubility in solvents.

A¹, A² and A³ each is preferably an atomic group represented by any one of the following formulae (L-II) to (L-V), in consideration of fluorescence intensity, regulation of excitation wavelength, regulation of affinity with the lanthanoid ion, ease of inclusion in the polymer fine particle and solubility in solvents.

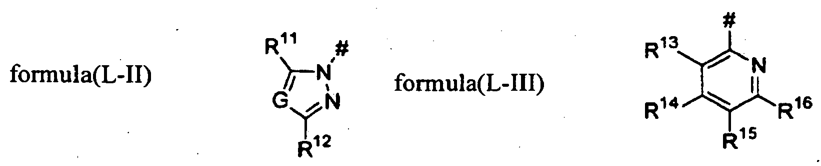

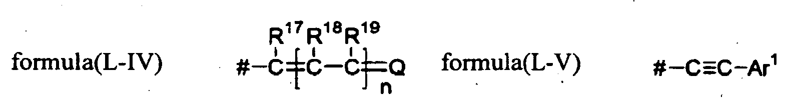

In the formulae, R¹¹ and R¹² each independently represent a hydrogen atom or a substituent. Examples of the substituent include an alkyl group, an aryl group, an amino group, a heterocyclic group, an alkylthio group, an arylthio group, an alkoxy group, an aryloxy group, an amide group, an ureido group and a halogen atom (fluorine, chlorine, bromine or iodine). In these examples, the substituent preferably contains, excluding hydrogen atoms, 1 to 50 atoms, more preferably 1 to 30 atoms and most preferably 1 to 20 atoms. In the case that the substituent contains an alkyl group, it may be of a cyclic structure or a chain-like structure which can be linear or branched, and may be saturated or may include an unsaturated bond. In the case that the alkyl group or aryl group has a substituent, preferable examples of the substituent include an alkyl group, an alkenyl group, an alkinyl group, an aryl group, a heterocyclic group, a silyl group, an alkoxy group, an amino group, an alkylamino group, a dialkylamino group, an acylamino group, an alkyl- or aryl-sulfonylamino group, an acyl group, an alkyl- or aryl-sulfonyl group, a formyl group, an alkoxycarbonyl group, a carbamoyl group, a sulfamoyl group, a halogen atom, a cyano group, a sulfo group and a carboxyl group.

R¹³ to R¹⁶ each independently represent a hydrogen atom or a substituent. In the case that any of R¹³ to R¹⁶ represents a substituent, preferable examples thereof include an alkyl group, an alkenyl group, an alkinyl group, an aryl group, a heterocyclic group, a silyl group, an alkoxy group, an amino group, an acylamino group, an alkyl- or aryl-sulfonylamino group, an acyl group, an alkyl- or aryl-sulfonyl group, a formyl group, an alkoxycarbonyl group, a carbamoyl group, a sulfamoyl group, a halogen atom (fluorine, chlorine, bromine or iodine), a cyano group, a sulfo group and a carboxyl group, each of which may be substituted when it can have a substituent. More preferable examples include an alkyl group, an alkenyl group, an alkinyl group, an aryl group, an acylamino group, a sulfonylamino group, a halogen atom and a cyano group, each of which may be substituted when it can have a substituent.

With respect to R¹³ to R¹⁶, a number of atoms, excluding hydrogen atoms, is preferably from 1 to 60, more preferably from 1 to 45 and most preferably from 1 to 35.

R¹⁷, R¹⁸ and R¹⁹ each independently represent a hydrogen atom or a substituent, and R¹⁷ and R¹⁸, R¹⁸ and R¹⁹ or R¹⁷ and R¹⁹ may be bonded with each other to form a ring. The substituent above means a substituent selected from a class of an alkyl group, an aryl group, a carboamide group, a sulfonamide group, an alkylthio group, a heterocyclic group, an alkoxy group, an aryloxy group and a combination thereof, and n represents 0, 1 or 2.

With respect to R¹⁷ to R¹⁹, a number of atoms, excluding hydrogen atoms, is preferably from 1 to 60, more preferably from 1 to 45 and most preferably from 1 to 35.

G represents a carbon atom or a nitrogen atom, and, in case of a carbon atom, may have a substituent. Examples of the substituent in this case are same as those cited for R¹¹ and R¹².

Q represents an atomic group required for forming a 5- or 6-membered nitrogen-containing heterocycle, and such nitrogen-containing heterocycle may constitute condensed rings. Q may also be bonded with R¹⁷, R¹⁸ or R¹⁹ to form a ring structure.

Preferable examples of heterocycle as Q are shown below. Following examples show heterocyclic skeletal structures, each of which may be utilized as a partially saturated skeleton and in which the position of hetero atom may be suitably selected in each cyclic structure. Condensed rings may be condensed at an arbitrary position. In addition, the preferable examples further include a cyclic structure represented by a combination of the following heterocycles.

That is, examples of heterocycle denoted by Q include pyrrole, pyrazole, imidazole, triazole, tetrazole, thiophene, furan, oxazole, thiazole, oxadiazole, thiadiazole, selenazole, pyridine, pyrimidine, pyrazine, pyridazine, triazine, tetrazine, oxazine, thiazine, oxadiazine, thiadiazine, pyrrolopyrrole, indole, pyrrolopyrazole, pyrroloimidazole, pyrrolotriazole, pyrrolotriazole, pyrrolotetrazole, thienopyrrole, pyrroloxazole, thienopyrrole, pyrroloxazole, pyrrolothiazole, pyrrolopyridine, pyrrolopyrimidine, pyrrolopyrazine, pyrrolopyridazine, pyrrolotriazine, pyrrolotetrazine, pyrroloxazine, pyrrolothiazine, pyrroloxazine, pyrrolothiadiazine, indazole, benzimidazole, benzotriazole, benzothiophene, benzofuran, benzoxazole, benzothiazole, benzoxadiazole, benzothiadiazole, benzoselenazole, quinoline, quinazoline, quinoxaline, phthalazine, benzotriazine, benzoxazine, benzothiazine, pyrazolopyrazole, pyrazoloxazole, pyrazolothiadiazole, pyrazolopyridine, pyrazolopyrimidine, pyrazolopyrazine, pyrazolopyridazine, pyrazolotriazine, pyrazoloxazine, pyrazolothiazine, pyrazolothiadiazine, imidazolopyrazole, pyrazolotriazole, pyrazolotetrazole, thienopyrazole, furopyrazole, pyrazoloxazole, imidazoloimidazole, imidazolotriazole, imidazolotetrazole, thienoimidazole, furoimidazole, imidazoloxazole, thienoimidazole, imidazoloxadiazole, imidazolothiadiazole, imidazoleselenazole, imidazolepyridine, imidazolopyrimidine, imidazolopyrazine, imidazolopyridazine, imidazolotriazine, imidazoloxazine, imidazolothiazine, imidazoloxadiazine, imidazolothiadiazine, triazolotriazole, thienotriazole, furotriazole, triazoloxazole, triazolothiazole, triazoloxadiazole, triazolothiadiazole, triazolopyridine, triazolopyrimidine, triazolopyrazine, triazolopyridazine, triazolotriazine, triazoloxazine, triazolothiazine, triazoloxadiazine, triazolothiadiazine, tetrazoloxazole, tetrazolothiazole, tetrazolopyridine, tetrazolopyrimidine, tetrazolopyrazine, tetrazolopyridazine, tetrazoloxazine, tetrazolothiazine, thienothiophene, thienofuran, thienoxazole, thienothiazole, thionoxadiazole, thienothiadiazole, thienoselenazole, thienopyridine, thienopyrimidine, thienopyrazine, thienopyridazine, thienotriazine, thienotetrazole, thienoxazine, thienothiazine, thienoxadiazine, thienothiadiazine, furoxazole, furothiazole, furoxadiazole, furothiadiazole, furopyridine, furopyrimidine, furopyrazine, furopyridazine, furotriazine, furoxazine, furothiazine, oxazoloxazole, thiazoloxazole, oxazoloxadiazole, oxazolothiadiazole, oxazolopyridine, oxazolopyrimidine, oxazolopyrazine, oxazolopyridazine, oxazolotriazine, oxazoloxazine, oxazolothiazine, oxazoloxadiazine, oxazolothiadiazine, thiazolothiazole, thiazoloxadiazole, thiazoloxadiazole, thiazoloselenazole, thiazolopyridine, thiazolopyrimidine, thiazolopyrazine, thiazolopyridazine, thiazolotriazine, thiazoloxazine, thiazolothiazine, thiazoloxadiazine, thiazolothiadizine, dithiole, dioxole, benzodithiole, and benzodioxole.

Q preferably contains, excluding hydrogen atoms, 4 to 70 atoms, more preferably 5 to 55 atoms and most preferably 6 to 45 atoms.

B¹ and B² each independently represent a nitrogen atom or =C(-R²⁰)-, wherein R²⁰ represents a hydrogen atom or a substituent. It is preferred that at least either of B¹ and B² represents a nitrogen atom.

R²⁰ represents a hydrogen atom or a substituent. In the case that R²⁰ represents a substituent, it is preferably an alkyl group, an alkenyl group, an alkinyl group, an aryl group, a heterocyclic group, a silyl group that may be substituted, an alkoxy group, an amino group, an acylamino group, a sulfonylamino group, an acyl group, a sulfonyl group, a formyl group, an alkoxycarbonyl group, a carbamoyl group, a sulfamoyl group, a halogen atom, a cyano group, a sulfo group or a carboxyl group, each of which may be substituted when it can have a substituent. More preferably R represents an alkyl group, an alkenyl group, an ankinyl group, an aryl group, an acyl group, a sulfonyl group, an alkoxycarbonyl group, a carbamoyl group, a halogen atom or a cyano group, each of these may be substituted when it can have a substituent. R²⁰ preferably contains, excluding hydrogen atoms, 1 to 30 atoms, more preferably 1 to 20 atoms and most preferably 1 to 10 atoms.

Ar¹ represents an aromatic carbon ring or an aromatic heterocycle, having 6 to 30 carbon atoms. Preferable examples of the aromatic carbon ring include a benzene ring, a thiophene ring, a furan ring, a pyrrole ring, a pyrazole ring, a triazole ring, a thiazole ring, an imidazole ring, an oxazole ring, an oxadiazole ring and a thiadiazole ring, and another ring may be further condensed to such ring structure. In the case that Ar¹ has a substituent, examples thereof are same as those cited for R¹¹ and R¹² above.

In the formulae (L-II) to (L-V), # indicates a position to be bonded with the nitrogen-containing heterocycle represented by the formula (L-I).

The nitrogen-containing heterocyclic ligand in the invention is, in consideration of the fluorescence intensity, absorption wavelength and ease of inclusion in the polymer microparticle, preferably those represented by the formula (L-VI), and more preferably those represented by the formula (L-VII). 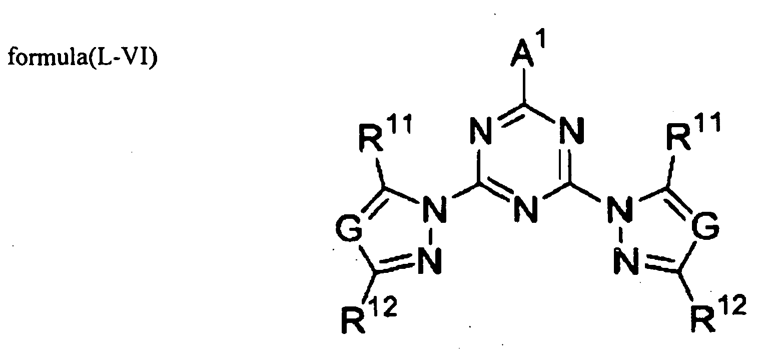 wherein A¹ has the same meaning as A¹ in the formula (L-I), and R¹¹, R¹² and G have the same meanings as R¹¹, R¹² and G in the formula (L-II). 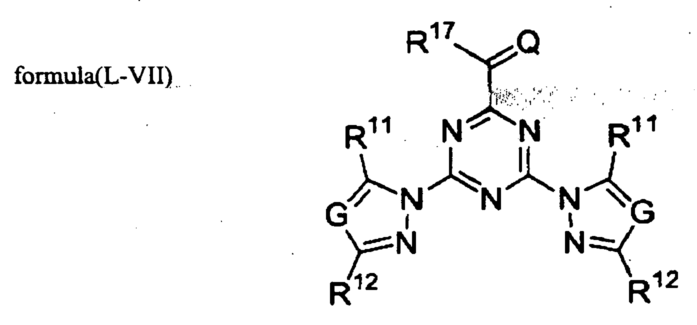 wherein R¹¹, R¹², R¹⁷, G and Q have the same meanings as R¹¹, R¹², R¹⁷, G and Q in the formulae (L-II) and (L-IV).

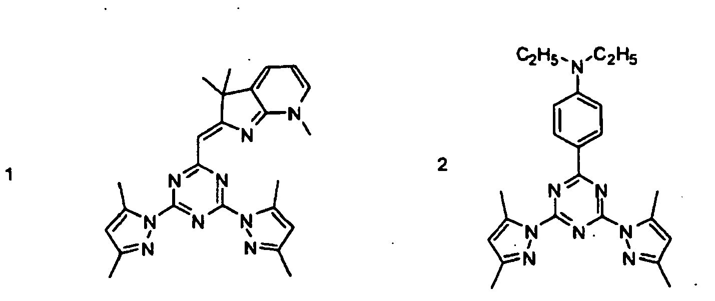

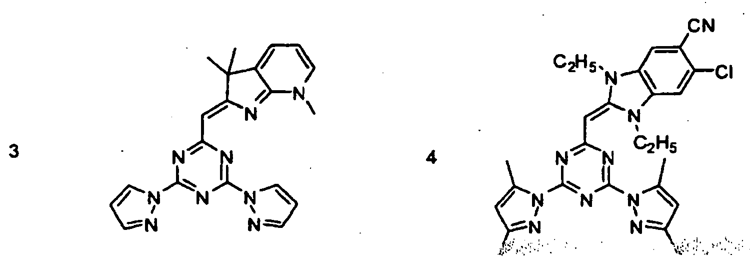

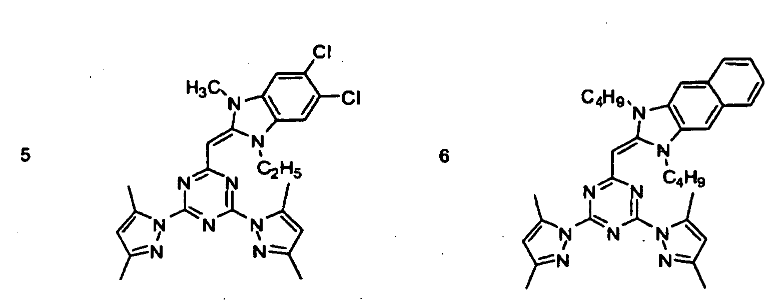

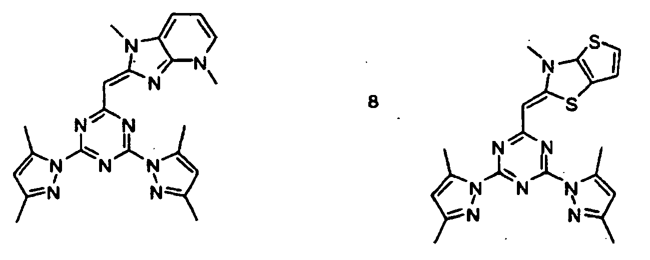

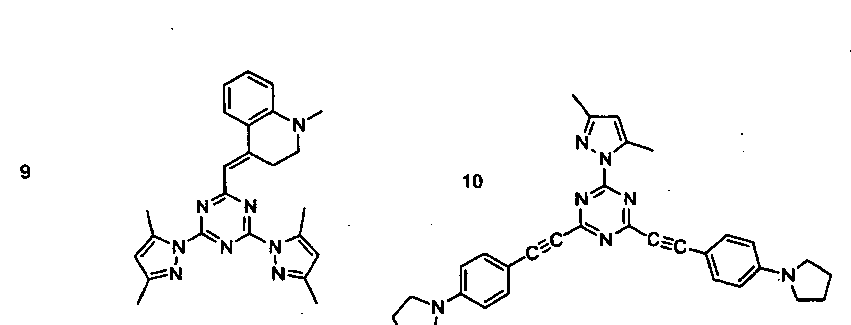

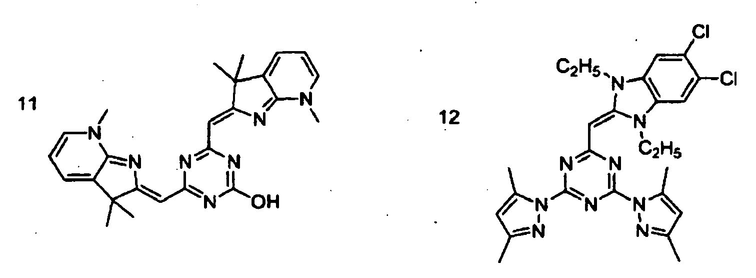

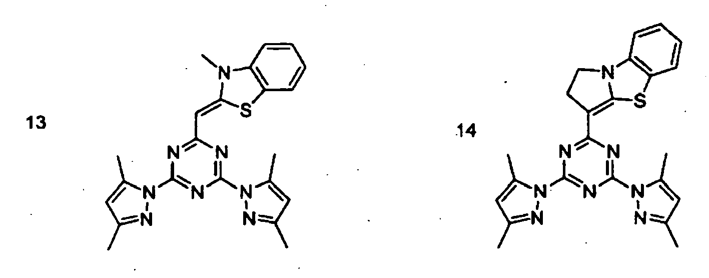

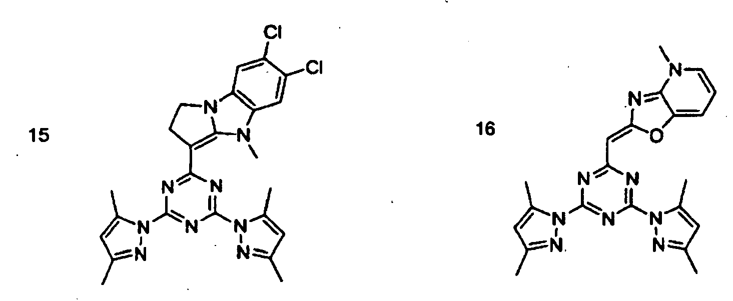

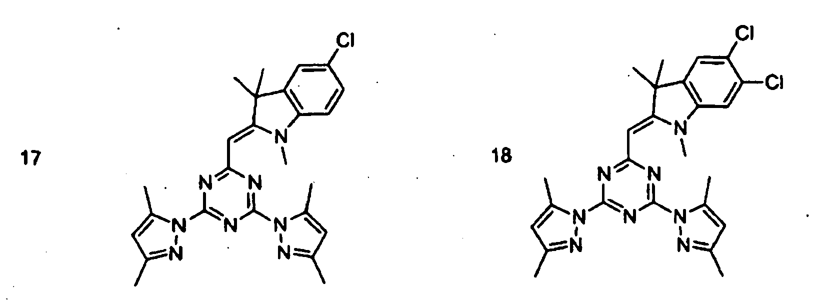

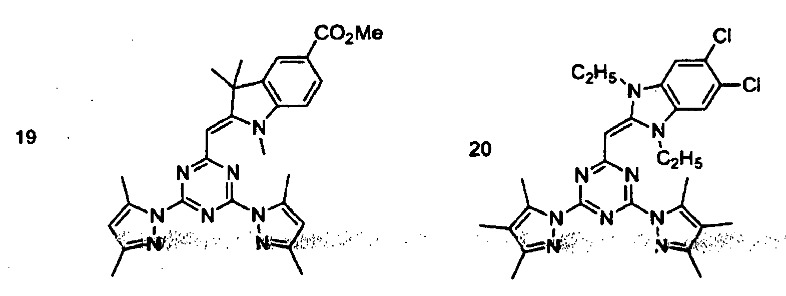

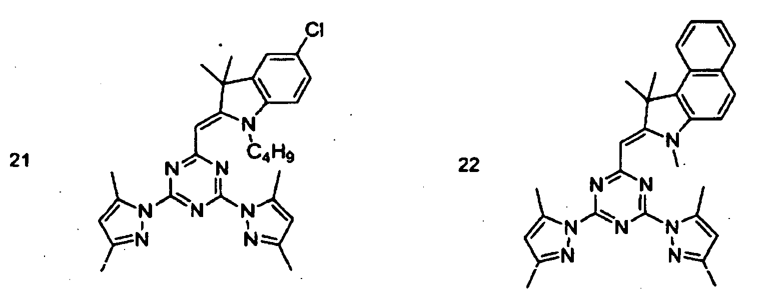

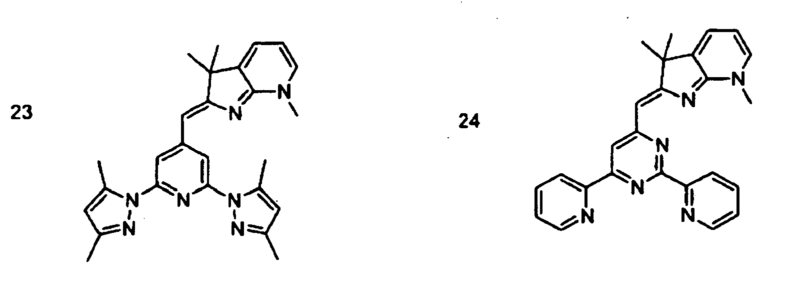

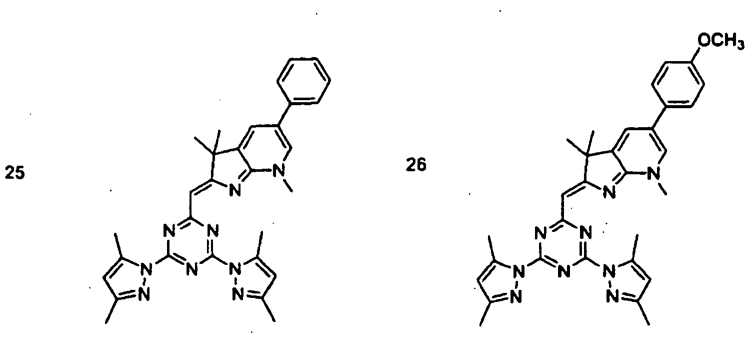

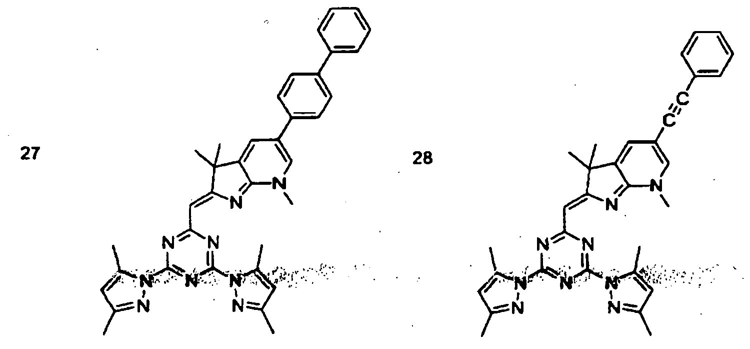

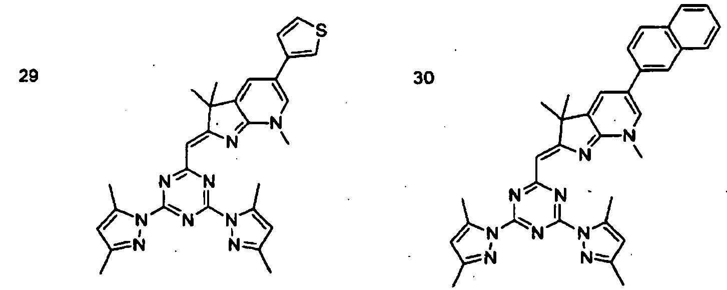

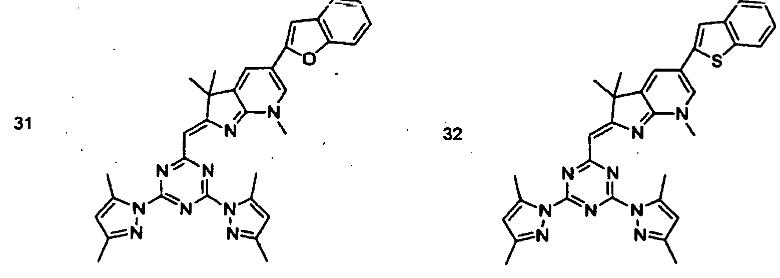

The example compounds above may be synthesized utilizing known compounds and known reaction conditions. The compound represented by the formula (I) may be synthesized preferably by a nucleophilic substitution reaction of the atomic groups A¹ to A³ on cyanuric acid chloride.

For example, it can be synthesized by reacting a compound represented by the formula (LP-I) and a compound represented by the formula (LP-III).

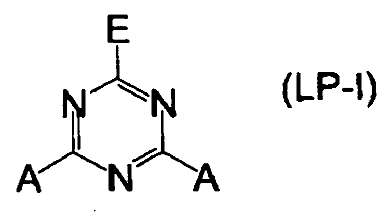

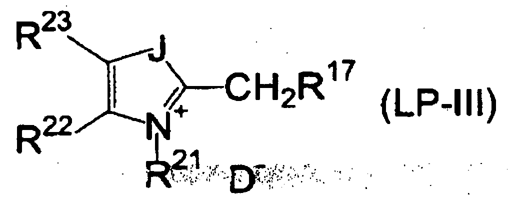

In the formula (LP-I), A is same as described above for A¹, A² and A³, and E represents a releasable group or A; and, in the formula (LP-III), R¹⁷ is same as described above, and R²⁰ represents an alkyl group that may be substituted or an aryl group (including heteroaryl group) that may be substituted, and that preferably has 1 to 30 carbon atoms, more preferably 1 to 20 carbon atoms and further preferably 1 to 12 carbon atoms.

R²² and R²³ each represent a hydrogen atom or a substituent, provided that R²¹ and R²² or R²² and R²³ may be bonded with each other to form a ring when possible. In the case that R²² and R²³ each represent a substituent, preferable examples thereof include an alkyl group, an aryl group, an amino group, a heterocyclic group, an alkylthio group, an arylthio group, an alkoxy group, an aryloxy group, an amide group, an ureido group and a halogen atom (fluorine, chlorine, bromine, or iodine). The substituent, in the examples cited above, preferably includes, excluding hydrogen atoms, 1 to 60 atoms, more preferably 1 to 45 atoms and most preferably 1 to 35 atoms.

D represents a counter ion for the ammonium ion in the formula (LP-III). J represents an oxygen atom, -C(R²⁴)(R²⁵)-, -N(R²⁶)- or a sulfur atom, and, may be bonded with R¹⁷, when possible, to form a ring. R²⁴, R²⁵ and R²⁶ each represent an alkyl group that may be substituted or an aryl group, and such alkyl group preferably has 1 to 30 carbon atoms, more preferably 1 to 20 carbon atoms and most preferably 1 to 10 carbon atoms, also may be of a cyclic structure or a chain-like structure which can be straight or branched, and may be saturated or may include an unsaturated bond.

Preferable examples of the releasable group as E in Formula (LP-1) include a halogen atom (fluorine, chloride, bromine or iodine), an aryloxy group (such as a phenoxy group or a 4-nitrophenoxy group), an arylthio group (such as a phenylthio group or a 4-bromophenylthio group), a sulfonyloxy group (such as a p-toluenesulfonyloxy group or a trifluoromethanesulfonyloxy group), a carbamoyloxy group (such as a dimethylcarbamoyloxy group or a morpholinocarbonyloxy group), among which a halogen atom is most preferable.

Examples of the counter ion as D in Formula (LP-III) include a halide ion (a fluorine ion, a chlorine ion, a bromine ion or an iodine ion), a sulfate ion, a perchlorate ion, a nitrate ion, a hydrosulfate ion, a sulfonate ion (such as a p-toluenesulfonate ion or a p-chlorobenzenesulfonate ion), a sulfate ester ion (such as a monomethyl sulfate ion), a tetrafluoroborate ion, and a hexafluorophosphate ion.

The nitrogen-containing heterocyclic ligand of the invention may be obtained by mixing a compound represented by Formula (LP-I) and a compound represented by Formula (LP-III), preferably in the presence of a solvent, and reacting these compounds preferably in the presence of a base at an appropriate temperature.

While most bases employed commonly in the organic syntheses are usable in the invention as the base in this operation, preferable examples thereof include pyridines (such as pyridine, or 2,6-lutidine), tertiary amines (such as triethylamine, N-ethyldiisopropylamine, N-methylmorpholine or tributylamine), guanidines (such as triphenylguanidine or 1,1,3,3-tetramethylguanidine), amidines (such as 1,8-diazabicyclo[5.4.0]-7-undecene, or 1,5-diazabicyclo[4,3,0]-5-nonene), anilines (such as N,N-diethylaniline), potassium carbonate, sodium carbonate, sodium hydrogencarbonate, sodium hydride, sodium methoxide, sodium ethoxide, sodium t-butoxide, potassium t-butoxide, potassium acetate, and sodium acetate, and more preferable examples thereof include pyridines, tertiary amines, guanidines and amidines.

While the solvent to be employed may be either a protonic solvent or a non-protonic solvent, a non-protonic solvent is preferable. Preferable examples thereof include acetonitrile, N,N-dimethylformamide, dimethyl sulfoxide, tetrahydrofuran, diethyl ether, N,N-dimethylacetamide, N-methylpyrrolidone, and 1,3-dimethyl-2-imidazolidinone.

While the reaction temperature should be suitably selected according to the respective reaction, in the present invention, it is generally within a preferable range of from -20° to 150°C, more preferably from 0° to 120°C and most preferably from 0° and 100°C.

While in the reaction, the materials may be charged in any order, it is preferable to dissolve or suspend the compound represented by Formula (LP-I) and the compound represented by Formula (LP-III) in the solvent, and the base is further added thereto under agitation.

### Synthesis examples

### Synthesis of Exemplary compound 4

For example, the Exemplary compound 4 may be synthesized by a following process.

18.4 g of cyanuric chloride are dissolved in 100 mL of dimethylacetamide, and 69.2 g of 3,5-dimethylpyrazole are added at the room temperature. Then the materials are reacted for 2 hours at a reaction temperature of 80°C.

After cooling, the reaction liquid is poured into water, and the deposited crystals are collected by filtration and recrystallized from dimethylformamide to obtain (LP-I-1) shown below. Obtained amount: 21.0 g, yield: 57.9 %.

NMR spectral data (in deuterized chloroform): 6.11 (3H, s), 2.81 (9H, s), 2.33 (9H, s).

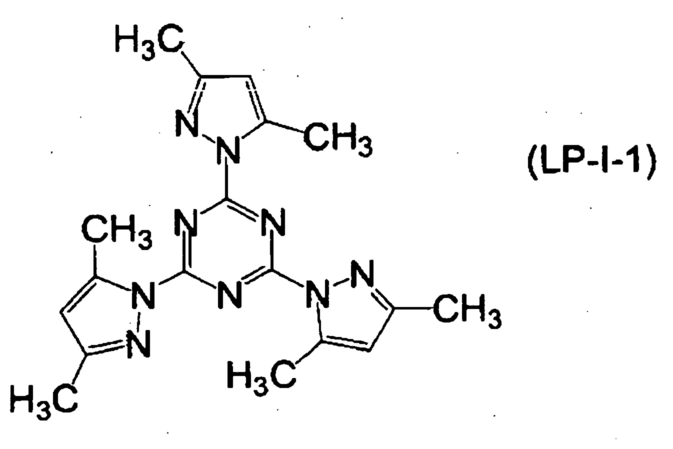

419 mg of 6-chloro-5-cyano-1,3-diethyl-2-methyl-1H-benzimidazolium p-toluenesulfonate and 363 mg of the aforementioned compound (LP-I-1) are suspended in 8 mL of dimethylsulfoxide, and, with an addition of 0.5 mL of tetramethylguanidine, are reacted for 30 minutes at 80°C.

After cooling, crystals deposited by addition of water are subjected to a filtration under a reduced pressure, and the obtained crystals are purified by a silica gel chromatography. The product is recrystallized from a mixture of methanol and ethyl acetate to obtain an object substance. Obtained amount: 287 mg, yield: 55.7%.

NMR spectral data (in deuterized chloroform): 7.34 (1H, s), 7.21 (1H, s), 6.04 (2H, s), 5.31 (1H, s), 4.53 (2H, q), 4.39 (2H, q), 2.70 (6H, s), 2.33 (6H, s), 1.31 (3H, t), 1.28 (3H, t).

Other Exemplary compounds can be synthesized in a similar manner as the Exemplary compound 4, by changing the portions corresponding to A¹ - A³ to those of the desired compound.

The nitrogen-containing heterocyclic ligand of the present invention may be employed in a range of 0.1 to 10 time equivalents with respect to the equivalent of the lanthanoid cation, and it may be employed more preferably in a range of 1 to 5 time equivalents.

Examples of the lanthanoid cation include cations of bivalent to tetravalent, and specific examples include Ce³⁺, Pr³⁺, Nd ³⁺, Nd⁴⁺, Sm²⁺, Sm³⁺, Eu²⁺, Eu³⁺, Tb³⁺, Dy³⁺, Dy⁴⁺, HO³⁺, Er³⁺, Tm²⁺, Tm³⁺, Yb²⁺ and Yb³⁺ . Among these, trivalent cations such as Pr³⁺, Nd³⁺, Sm³⁺, Eu³⁺, Tb³⁺, Dy³⁺, HO³⁺, Er³⁺, Tm³⁺, and Yb³⁺ are preferable as they emit fluorescence with features of a region from ultraviolet to near infrared, a long lifetime, a narrow wavelength width and the like, particularly Nd³⁺, Sm³⁺, Eu³⁺, Tb³⁺, Dy³⁺ and Tm³⁺ are more preferable, and Eu³⁺ and Tb³⁺ are most preferable in terms of the fluorescence intensity.

In stead of the ligand of Formula (L-I), or in combination with the ligand of Formula (L-I), other known organic ligands for lanthanoid cations may be used, in view of the fluorescence intensity and the ease of synthesis of lanthanoid dyes.

Examples of other organic ligands include aromatic amines (Helv. Chim. Acta., Vol. 79, P.789, 1966), β-diketones (Anal. Chem., Vol. 70, P.596-601, 1998), and aromatic group-containing carboxylic acids (Chem. Mater., Vol. 10, 286-296, JP-A-2000-345052). Specific examples include 4,4,4-trifluoro-1-(2-thienyl)-1,3-butanedione, 4,4,4-trifluoro-1-phenyl-1,3-butanedione, and 4,4,4-trifluoro-1-(2-naphthyl)-1,3-butanedione. Examples of aromatic carboxylic acid include dendrone having a carboxylate group in a focal point and including an aromatic ring in a repeating unit.

In combination with such ligands, there may be employed, for the purpose of suppressing a loss in the fluorescence intensity of the lanthanoid dye, phosphineoxides, phosphate esters, sulfoxides, phisphite esters, phosphines, sulfides, amines and aromatic nitrogen-containing heterocyclic compounds.

A fluorescent lanthanoid complex, namely the fluorescent lanthanoid dye of the invention, may be easily synthesized by adding a ligand solution, containing at least the ligand of the invention, to a solution containing a lanthanoid element. When the fluorescent lanthanoid complex is deposited from the solution, it can be separated by filtration. When the fluorescent lanthanoid complex is not deposited from the solution, crystals may be obtained by distilling off the solvent, and purified if necessary for use.

While a concentration of the fluorescent lanthanoid complex in the polymer fine particle of the invention is not restricted, it is normally in a range of 0.01 to 50 mass% with respect to the mass of the polymer fine particle, preferably in a range of 0.05 to 20 mass% in consideration of the light intensity, and more preferably in a range of 0.1 to 10 mass%.

### <Production method of fluorescent polymer fine particle>

The fluorescent polymer fine particle of the present invention can be produced by introducing (incorporating) a lanthanoid fluorescent dye in the fine particle, prepared by various known methods explained above.

The dye introduction can be executed in the following manner. The fine particles and the lanthanoid fluorescent dye are immersed in a solution, containing an organic solvent, capable of swelling the water-insoluble polymer weight compound constituting the hydrophobic portion of the fine particles (organic solvent being for example acetone or toluene), at a predetermined proportion. Thus the water-insoluble polymer weight compound is caused to swell, and along with such swelling, the lanthanoid fluorescent dye is incorporated into the fine particles. Then the organic solvent is removed from the mixture, whereby the water-insoluble polymer weight compound is caused to shrink, but the lanthanoid fluorescent dye of hydrophobic property is unable to come out of the fine particle and is sealed. If desired, the lanthanoid fluorescent dye that has not been incorporated in the fine particle is removed, whereby the fluorescent polymer fine particle of the invention can be obtained.

In the producing method above, the method of removing the organic solvent from the mixture is not particularly limited, and is for example a method of evaporating the organic solvent to dry by an evaporator or the like, or a method of causing a shrinkage in a non-solvent (for example a method of replacing the organic solvent-containing solution with a solution not containing the organic solvent).

Also the proportion of the organic solvent, in the organic solvent-containing solution to be used for swelling the microparticle, is not particularly limited so far as the water-insoluble polymer weight compound can be swelled to an extent that the lanthanoid fluorescent dye is incorporated in the water-insoluble polymer compound, but it may be within a range of from 2 to 50 (vol/vol) %.

In addition to the foregoing, there may also be adopted a method of dissolving the lanthanoid fluorescent dye in a liquid containing the polymerizable monomer and the macromonomer, followed by a polymerization thereby incorporating the lanthanoid fluorescent dye simultaneously with the formation of the fine particle.

In the case that the hydrophilic macromonomer of the invention contains a reactive functional group, the method further includes an activation of such reactive functional group. The method of such activation may be suitably selected according to the type of the reactive functional group unit.

For example, when the reactive functional group unit contains an amino group precursor structure, a treatment with an acid or an alkali is executed on the polymer fine particle, in order to convert the amino group precursor structure into an amino group. The acid may be an inorganic acid such as hydrochloric acid, sulfuric acid or nitric acid, or an organic acid such as p-toluenesulfonic acid or acetic acid. A pH is preferably 5 or less, and more preferably 3 or less. The alkali may be an aqueous sodium hydroxide solution or an aqueous potassium hydroxide solution, and a pH is preferably 9 or higher, and more preferably 11 or higher. A reaction temperature is preferably from 25 to 100°C, more preferably from 40 to 90°C.

Such activating treatment may be conducted, depending on the specific treatment to be executed, before or after the step for introducing the fluorescent lanthanoid dye.

### <Fluorescence detecting complex member>

A complex for detecting fluorescence of the invention is constituted of a fluorescent polymer fine particle of the invention and a combination substance capable of binding the fluorescent polymer fine particle and a target substance to be detected. Thus the target substance can be detected efficiently and with a high sensitivity, based on a fluorescence emission from the fluorescent polymer fine particle combined through the combination substance.
Examples of the combination material include an antigen and an antibody. Examples of such antibody include IgG and IgM of polyclonal antibodies of a rabbit or a goat, or of monoclonal antibodes of a mouse, and F(ab')₂, Fab and Fab' fractions obtained by an enzyme treatment or a reducing treatment thereof. Also examples of the antigen include various materials such as proteins, polypeptides, steroids, polysaccharides, lipids, drugs and pollens.

Examples of the method of bonding such antibody or antigen include a method of bonding a sugar chain of the antibody or the antigen to an amino group of the polymer fine particle by periodic acid, a method of bonding an amino group of the antibody or the antigen to an amino group of the polymer fine particle by glutaraldehyde, and a method of introducing a maleimide group to an amino group of the polymer fine particle by a reaction with Sulfo-SMCC (sulfosuccinimidyl 4-(N-maleimidemethyl)cyclohexane-1-carboxylate) and bonding it with a mercapto group of the antibody. A bonding amount, in a mass per 1 milligram of the polymer fine particles, is normally from 50 nanograms to 500 micrograms, preferably from 500 nanograms to 200 micrograms. Thus the complex member is usable as an immunoanalysis reagent having a fluorescent property (fluorescent immunoanalysis reagent).

Examples of the combination substance further include, in addition to the antigen and the antibody, allergens, enzymes, enzyme substrates, coenzymes, enzyme inhibitors, host compounds, hormones, hormone receptors, proteins, blood proteins, tissue proteins, cells, cell fragments, karyoplasms, viruses, virus particles, metabolites, neurotransmitters, haptens, drugs, nucleic acids, metals, metal complexes, microorganisms, parasites, bacteria, biotins, avidins, lectins, sugars, physiologically active substances, physiologically active substance receptors, environmental substances, chemical species and modified compounds thereof, according to the target substance of detection. For example, in the case where the target substance of detection can be treated with avidin in advance, biotin may be selected as the combination substance. Also in the case where the target substance of detection is a certain ligand, a receptor or a fragment thereof, which specifically bond to such ligand, may be selected. Also in the case where the target substance of detection is a nucleic acid, a protein or a peptide (aptamer) bonding specifically to the nucleic acid may be selected.

The bonding with such combination substance may be executed as described above, or may be executed by other known bonding methods.
Such combination substance may be the target substance to be detected, or may be rendered detectable by the complex for detecting fluorescence, by a bonding treatment to the target substance of detection in a similar manner to the fluorescent polymer fine particle.

### [Fluorescence detecting complex member set]

The fluorescence detecting complex member set of the present invention is a set including at least a first fluorescence detecting complex member, which is constituted of a first fluorescent polymer fine particle contained in the fluorescent polymer fine particle set, and of a first binding material capable of binding the fluorescent polymer fine particle and the target substance of detection, and a second fluorescence detecting complex member, which is constituted of a second fluorescent polymer fine particle contained in the fluorescent polymer fine particle set, and of a second binding material different from the first binding material.

As the different combination substances are bonded to the respectively different fluorescent polymer fine particles, the fluorescent polymer fine particles respectively corresponding to the combination substances can be detected separately in a single operation, with high sensitivities.

Also the use of the fluorescence detecting complex member allows to detect the target substance of detection in a simpler manner, without requiring a step of forming the fluorescence detecting complex member from the fluorescent polymer fine particle and the combination substance.

In the fluorescence detecting complex member of the invention, the aforementioned matters relating to the fluorescence detecting complex member are applicable.

In the present invention, a target substance of detection for the first binding material and a target substance of detection for the second binding material are preferably different from each other. In this manner, different target substances of detection can be detected separately in a single operation, with high sensitivities.

In the present invention, it is also preferable that the combination substances have a same target substance of detection, and that the first binding material and the second binding material are different in detection sensitivities. In this manner, the target substance of detection can be detected quantitatively over a wider sensitivity range.

Also the fluorescence detecting complex member set of the invention may further include at least a third fluorescence detecting complex member, which is constituted of first and second fluorescent polymer fine particles and a third combination substance different from the first and second binding materials. In this manner, the target substances of detection of arbitrary types and a wider sensitivity range can be detected separately in a single operation, with high sensitivities.

### [Use of the fluorescent polymer fine particle set]

The fluorescent polymer fine particle set of the present invention includes at least a first fluorescent polymer fine particle containing a polymer fine particle having a core-shell configuration formed by a hydrophobic core and a hydrophilic shell, and a first fluorescent lanthanoid dye incorporated in the polymer fine particle and containing a lanthanoid cation, and a second fluorescent polymer fine particle containing the polymer fine particle, and a second fluorescent lanthanoid dye different from the first fluorescent lanthanoid dye.

In this manner, plural target substances of detection can be detected separately in a single operation, with high sensitivities. Also, such fluorescent polymer fine particle set can generate fluorescences of plural light emission wavelengths by an excitation energy source of a single excitation wavelength, and is used in a display apparatus, an illumination apparatus and a decoration apparatus.

In the fluorescent polymer fine particle of the invention, matters on known fluorescent polymer fine particle are applicable.

Also the fluorescent polymer composition of the invention may further include at least a third fluorescent polymer fine particle, which is constituted of the polymer fine particle, and a third fluorescent lanthanoid dye, different from the first and second fluorescent lanthanoid dyes. In this manner, there can be obtained a fluorescent polymer fine particle composition, having arbitrary excitation wavelength/light emission wavelengths.

In the fluorescent polymer fine particle composition of the present invention, it is preferable that the first fluorescent polymer fine particle constitutes a first fluorescence detecting complex member, together with a first binding material capable of binding the fluorescent polymer fine particle with a target substance of detection, and that the second fluorescent polymer fine particle constitutes a second fluorescence detecting complex member, together with a second binding material different from the first binding material. In this manner, plural target substances of detection can be detected separately in a single operation, with high sensitivities.

Also the fluorescent polymer set of the invention may further include at least a third fluorescence detecting complex member, which is constituted of a third fluorescent polymer fine particle containing the polymer fine particle and a third fluorescent lanthanoid dye different from the first and second fluorescent lanthanoid dyes, and a third combination substance different from the first and second binding materials. In this manner, the target substances of detection of arbitrary types can be detected separately in a single operation, with high sensitivities.

In the fluorescence detecting complex member of the invention, the aforementioned matters relating to the fluorescence detecting complex member are applicable.

### [Method of detecting fluorescence]

The fluorescence detecting method of the invention includes a step of forming a first fluorescence detecting complex member from a first fluorescent polymer fine particle contained in the fluorescent polymer fine particle set of the invention and a first binding material capable of binding the fluorescent polymer fine particle and a target substance of detection, a step of forming a second fluorescence detecting complex member from a second fluorescent polymer fine particle contained in the fluorescent polymer fine particle set of the invention and a second binding material different from the first binding material, a step of bringing the first and second fluorescent detecting complex members into contact with a specimen containing the target substance of detection, a first detection step of detecting the first fluorescent lanthanoid dye contained in the first fluorescence detecting complex member, and a second detection step of detecting the second fluorescent lanthanoid dye contained in the second fluorescence detecting complex member.

The construction above enables selection of the combination substance according to the necessity. It is thus possible to construct a fluorescence detecting complex member set corresponding to the target substance of detection more efficiently, and to detect the target substance of detection more efficiently.

In the fluorescence detecting complex member of the invention, the aforementioned matters on the fluorescence detecting complex member are applicable.

Also the fluorescence detecting method of the present invention is a method including at least a step of bringing the fluorescence detecting complex member contained in the fluorescence detecting complex member set into contact with a specimen containing the target substance of detection, a first detection step of detecting the first fluorescent lanthanoid dye contained in the first fluorescence detecting complex member, and a second detection step of detecting the second fluorescent lanthanoid dye contained in the second fluorescence detecting complex member. In the present invention, as the fluorescence detecting complex member is constructed in advance, it is possible to detect the target substance of detection in simpler manner.

The fluorescence detecting method of the present invention includes detection of a target substance of detection, corresponding to a combined substance of the fluorescence detecting complex member, utilizing a mixture of two or more fluorescence detecting complex members. In this manner, plural target substances of detection can be detected in a single operation, with high sensitivities.

In the fluorescence detecting method, the procedure and conditions of the known fluorescence detecting method utilizing a fluorescent substance may be applicable, and the detecting method includes a preparation of a specimen for detection by contacting the target substance of detection with the fluorescence detecting complex member, an irradiation with an excitation light for exciting the fluorescence detecting complex member, and a measurement of the fluorescence emission caused by such irradiation.

The fluorescence detecting method of the present invention, in the case that the fluorescent lanthanoid dyes contained in two or more fluorescence detecting complex members have different excitation wavelengths, includes at least a first detection step of executing an irradiation with an excitation light of an excitation wavelength corresponding to the first fluorescent lanthanoid dye and a measurement of a fluorescence emission induced by the irradiation, and a second detection step of executing an irradiation with an excitation light of an excitation wavelength corresponding to the second fluorescent lanthanoid dye and a measurement of a fluorescence emission induced by the irradiation.

According to the present invention, the method may further include at least a third detection step of executing an irradiation with an excitation light of an excitation wavelength corresponding to the third fluorescent lanthanoid dye and a measurement of a fluorescence emission induced by the irradiation.

Also the fluorescence detecting method of the present invention, in the case that the fluorescent lanthanoid dyes contained in two or more fluorescence detecting complex members have different light emission wavelengths, includes at least a first detection step of executing an irradiation with an excitation light of a corresponding excitation wavelength and a measurement of a fluorescence emission derived from the first fluorescent lanthanoid dye and induced by the irradiation, and a second detection step of executing a measurement of a fluorescence emission derived from the second fluorescent lanthanoid dye and induced by the irradiation.

Furthermore, in the present invention, the method may further include at least a third detection step of executing an irradiation with an excitation light of an excitation wavelength corresponding to the fluorescent lanthanoid dyes and a measurement of a fluorescence emission derived from the third fluorescent lanthanoid dye and induced by the irradiation.

Furthermore, in the present invention, it is possible to measure simultaneously the fluorescence emissions of different light emission wavelengths derived from the respective fluorescent lanthanoid dyes, or to execute the irradiation of the excitation light and the measurement of the emitted fluorescence, in succession corresponding to the respective fluorescence detecting complex members.

Also the fluorescence detecting method of the invention, utilizing a lanthanoid complex (particularly of europium, terbium or ruthenium) of a long emission lifetime as the fluorescent dye, is preferably a delayed fluorescence analysis in which a target fluorescence is detected after the extinction of a background fluorescence. For example, an europium complex has an emission lifetime in the order of several hundred microseconds to a millisecond, which is 100,000 to 1,000,000 times of that of the ordinary organic dyes. Besides, it has features of a large Stokes shift equal to or larger than 250 nm and a sharp fluorescent peak. The analysis is executed by exciting the ligand of the lanthanoid ion, and measuring an induced delay by a time-resolved fluorescence measuring apparatus.

Also the fluorescent lanthanoid dye in the invention can be excited with a visible light, which is absorbed by the nitrogen-containing heterocyclic ligand, for example a light of from 400 to 500 nm. It is therefore unnecessary to use an excitation light of a high energy such as in the ultraviolet region, and the selection of the excitation light source has a larger freedom.

Thus a fluorescence detection of a high sensitivity can be realized with an excitation light source of a low energy.

The fluorescence detecting method of the invention is more preferably executed by a time-resolved fluorescence measurement, in consideration of precision and sensitivity of the measurement. Conditions of the time-resolved fluorescence measurement may be suitably selected according to the selected metal ion.

### <Fluorescence detecting kit>

A fluorescence detecting kit may be constructed utilizing the fluorescent polymer fine particle set of the present invention. Thus the target substance can be detected efficiently and with a high sensitivity, based on a fluorescence emission from the fluorescent polymer fine particle based on the combination substance.

In the fluorescence detecting kit, the fluorescent polymer fine particle may be contained together with the combination substance, or may be contained as the fluorescence detecting complex member.

The kit may further contain a reagent for rendering the target substance of detection detectable by the complex for detecting fluorescence, according to the kind of the combination substance. Examples of such reagent include, in case of selecting a biotin as the combination substance in the complex for detecting fluorescence, a reagent for avitinizing the target substance to be detected.

### EXAMPLES

In the following, the present invention will be clarified further by examples.

### Example 1

### <Synthesis of hydrophilic macromonomer MM-31>

Under a nitrogen flow, 42.56 g of N-vinylacetamide and 5.31 g of mercaptopropionic acid were dissolved in 127 g of ethanol, and heated to 60°C. Then 2.48 g of 2,2'-azobis(2,4-dimethylvaleronitrile) were added, and the mixture was agitated for 4 hours at 60°C and further for 2 hours at 75°C. After cooling to the room temperature, the reaction mixture was diluted by adding 50 g of ethanol. Then the mixture was poured into 2L of ethyl acetate, and a precipitated polymer was separated by suction filtration and dried under vacuum at the room temperature. Thus 34.7 g of polymer were obtained. In an acid value measurement by a titration with a 0.1 mol/L aqueous solution of potassium hydroxide, the polymer contained acid of 0.29 mmols per 1 g. Also according to a GPC measurement, this polymer had a number-average molecular weight Mn of 3,500.

Then 33 g of the polymer obtained above were dissolved in 132 g of dimethylsulfoxide, then added with 15.1 g of 2-hydroxyethyl methacrylate, 0.39 g of N,N-dimethylaminopyridine and 7.3 g of N,N-diisopropylcarbodiimide, and the mixture was agitated at 40°C for 4 hours. After cooling to the room temperature, it was diluted by adding 30 g of ethanol. Then the mixture was poured into 2L of ethyl acetate, and a precipitated polymer was separated by suction filtration and dried under vacuum at the room temperature to obtain 31.3 g of macromonomer MM-31. In a titration with a 0.1 mol/L aqueous solution of potassium hydroxide, no acid was detected, indicating that carboxylic acid in the polymer vanished by a reaction with hydroxyethyl methacrylate. Also according to a GPC measurement, the hydrophilic macromonomer MM-31 had a number-average molecular weight of 3,600.

### <Synthesis of fluorescent fine particle dispersion-1>

3.6 g of thus synthesized hydrophilic macromonomer MM-31 (number-average molecular weight: 3,600) were dissolved in a mixture of 32 g of distilled water and 10 g of ethanol, and were heated to 60°C under a nitrogen flow of 50 mL per minute. In this solution, a solution formed from 1 g of methyl methacrylate, 0.03 g of 2,2'-azobis(2,4-dimethylvaleronitrile) and 10 g of ethanol was added dropwise over a period of 6 hours. After the completion of dropwise addition, the mixture was agitated at 70°C for 3 hours, further at 75°C for 2 hours and cooled to the room temperature. The dispersion was purified by a dialysis (fractionated molecular weight: 12,000 - 14,000) with distilled water for one day. The particles were separated by a centrifuge, and diluted and re-dispersed in distilled water to a particle concentration of 5 mass%. A volume-average particle size, measured with a particle size distribution measuring apparatus (Coulter N4Plus, manufactured by Beckman-Coulter Inc.), was 350 nm.

After hydrochloric acid was added to pH = 1 to the particle dispersion, the mixture was agitated at 95°C for 24 hours to hydrolyze acetamide group, in the hydrophilic polymer, bonded to the particle surface, thereby converting it into an amino group. The dispersion was cooled to the room temperature, then adjusted to pH = 7, further purified by a dialysis with distilled water for one day for removing the water-soluble substances, and the particles were separated by a centrifuge, and subjected to a concentration adjustment with distilled water to obtain a 4 mass% dispersion liquid A of fine particles having amino groups on the surface.

Then 7.0 mg of tris(4,4,4-trifluoro-1-(2-thienyl)-1,3-butanediono)europium (III) and 4.5 mg of the ligand having a triazine ring of exemplary compound 12 were dissolved in 0.5 g of methanol. Then the solution was added to 10 g of the dispersion liquid A of the above-synthesized fine particles having amino groups on the surface, and agitated at the room temperature for 3 hours. Thereafter, methanol in the dispersion liquid was distilled off under a reduced pressure, utilizing an aspirator. After the undissolved substance was filtered off, the dispersion was let to stand at 45°C for 3 days, and then cooled to the room temperature to obtain a fluorescent fine particle dispersion liquid-1. The fluorescent fine particle dispersion liquid-1 showed a strong fluorescence at 615 nm by an irradiation with an excitation light of 420 nm.

### <Synthesis of Antibody-combined fine particle 1>

Then an antibody Fab' was introduced into the fluorescent polymer fine particles synthesized above by an ordinary hinge method (Eiji Ishikawa et al., "Enzyme Immunomeasurement, 3rd ed.", Igaku Shoin).

The thus-synthesized fluorescent polymer fine particles dyed with the europium dye, having amino groups on the surface thereof, were dispersed in a 0.1M HEPES buffer (pH 8.0) so that a concentration thereof became 14 mg/mL. 0.6 mg of Sulfo-GMBS (available from Dojin Chemical Co.) were added to 1 mL of the fine particle dispersion and were reacted at the room temperature for 1 hour, and the reaction product was purified by a column (trade name: PD-10, manufactured by Pharmacia Bioscience Inc.) to obtain a dispersion of fluorescent polymer fine particles having maleimide groups on the surface.

Then, 3.9 mg of a Fab' fraction, purified from 7.2 mg of anti-theophylline antibody (Cosmo Bio Co.) by a pepsin treatment and a reduction with mercaptoethylamine, were dissolved in a O.1M HEPES buffer (pH 7.4) in such a manner that a concentration thereof became 1 mg/ml. It was mixed with an equal amount of a solution (1 mg/ml in 0.1M HEPES (pH 7.4)) of fluorescent fine particles having maleimide groups on the surface, then the mixture was agitated overnight at 4°C and purified by gel filtration to obtain antibody-bonded fluorescent fine particles-1.

### <Synthesis of fluorescent fine particle dispersion-2>

Then 7.0 mg of tris(4,4,4-trifluoro-1-(2-thienyl)-1,3-butanediono)europium (III) and 4.6 mg of the ligand having a triazine ring of exemplary compound 28 were dissolved in 0.5 g of methanol. Then the solution was added to 10 g of the dispersion liquid A of the above-synthesized fine particles having amino groups on the surface, and agitated at the room temperature for 3 hours. Thereafter, methanol in the dispersion liquid was distilled off under a reduced pressure, utilizing an aspirator. After the undissolved substance was filtered off, the dispersion was left to stand at 45°C for 3 days, and then cooled to the room temperature to obtain a fluorescent fine particle dispersion liquid-2. The fluorescent fine particle dispersion liquid-2 showed a strong fluorescence at 615 nm by an irradiation with an excitation light of 480 nm.

### <Synthesis of antibody-combined fine particle-2>

The thus-synthesized fluorescent fine particle dispersion-2 was dispersed in a 0.1 M HEPES buffer (pH 8.0) in such a manner that a concentration thereof became 14 mg/ml. 0.6 mg of Sulfo-GMBS (available from Dojin Chemical Co.) were added to 1 mL of the fine particle dispersion and were reacted at the room temperature for 1 hour, and the reaction product was purified by a column (trade name: PD-10, manufactured by Pharmacia Bioscience Inc.) to obtain a dispersion of fluorescent fine particles having maleimide groups on the surface.

Then, 4.0 mg of a Fab' fraction, purified from 10 mg of anti-folic acid antibody (Cosmo Bio Co.) by a pepsin treatment and a reduction with mercaptoethylamine, were dissolved in a 0.1M HEPES buffer (pH 7.4) in such a manner that a concentration thereof became 1 mg/ml. It was mixed with an equal amount of a solution (1 mg/ml in 0.1M HEPES (pH 7.4)) of fluorescent fine particles having maleimide groups on the surface, then the mixture was agitated overnight at 4°C and purified by gel filtration to obtain antibody-combined fluorescent fine particles-2.

### <Preparation of Theophylline-bonded poly-L-lysine, Bonding thereof to a quartz substrate and Detection thereof >

40 mL of 0.1% poly-L-lysine (available from Sigma Co.) and 10 mL of 1M MES (pH 6.0) were mixed, and 35 mg of theophylline-8-butanoic acid (available from Sigma Co.), 30 mg of WSC (available from Dojin Chemical Co.) and 34 mg of Sulfo-NHS (available from Pierce Co.) were added and reacted at the room temperature for 6 hours. The reaction liquid was subjected to a gel filtration utilizing a gel filtration column (trade name: SEPHADEX^{®} G-25, manufactured by Pharmacia Bioscience Inc.) to purify theophylline-bonded poly-L-lysine. A synthetic quartz substrate, subjected to an alkaline surface treatment, was sufficiently rinsed with purified water, and was immersed in the solution of theophylline-bonded poly-L-lysine at the room temperature for 2 hours. It was then rinsed with purified water and air dried for use in the following experiments.

### <Preparation of folic acid-bonded poly-L-lysine, bonding thereof to a quartz substrate>

40 mL of 0.1% poly-L-lysine (available from Sigma Co.) and 10 mL of 0.1 M MES (pH 6.0) were mixed, and 65 mg of folic acid (available from Wako Pure Chemical Industries Co.), 30 mg of WSC (available from Dojin Chemical Co.) and 34 mg of Sulfo-NHS (available from Pierce Co.) were added and reacted at the room temperature for 6 hours. The reaction liquid was subjected to a gel filtration utilizing Cefadex G-25 to purify folic acid-bonded poly-L-lysine. A synthetic quartz substrate, subjected to an alkaline surface treatment, was sufficiently rinsed with purified water, and was immersed in the solution of folic acid-bonded poly-L-lysine at the room temperature for 2 hours. It was then rinsed with purified water and air dried for use in the following experiments.

### <Detection>

The antibody-combined fluorescent fine particles 1 and 2 synthesized above were diluted to a fine particle concentration of 0.001 mass % and were mixed in equal amounts. 1 µl of the mixed fine particles was spotted on each of the quartz substrate bonded with theophylline-bonded poly-L-lysine and the quartz substrate bonded with folic acid-bonded poly-L-lysine, each of which was then let to stand at the room temperature for 2 hours, and rinsed with purified water. After the water drops on the surface were removed, the quartz substrate was irradiated with lights of 420 and 480 nm and subjected to a fluorescence measurement in a time-resolved mode. As a result, in the quartz substrate bonded with theophylline-bonded poly-L-lysine, a strong fluorescence was observed by the irradiation with the light of 420 nm, but scarce fluorescence was observed in the irradiation with the light of 480 nm. In the quartz substrate bonded with folic acid-bonded poly-L-lysine, scarce fluorescence was observed by the irradiation with the light of 420 nm, but a strong fluorescence was observed in the irradiation with the light of 480 nm. These results indicate that theophylline and folic acid can be independently detected with detection lights of a same wavelength, by utilizing a fine particle mixed liquid and by changing the wavelength of the excitation light.

### Example 2

### <Synthesis of fluorescent fine particle dispersion-3>

A dispersion liquid A of fine particles having amino groups on the surface was synthesized in the same manner as in Example 1. Then 7.0 mg of tris(4,4,4-trifluoro-1-(2-thienyl)-1,3-butanediono)europium (III) and 4.5 mg of the ligand having a triazine ring of exemplary compound 5 were dissolved in 0.5 g of methanol. Then the solution was added to 10 g of the dispersion liquid A of the above-synthesized fine particles having amino groups on the surface, and agitated at the room temperature for 3 hours. Thereafter, methanol in the dispersion liquid was distilled off under a reduced pressure, utilizing an aspirator. After the undissolved substance was filtered off, the dispersion was let to stand at 45°C for 3 days, and then cooled to the room temperature to obtain a fluorescent fine particle dispersion liquid-3. The fluorescent fine particle dispersion liquid-3 showed a strong fluorescence at 615 nm by an irradiation with an excitation light of 410 nm.

### <Synthesis of antibody-combined fine particle-3>

The thus-synthesized fluorescent fine particle dispersion-3 was dispersed in a 0.1M HEPES buffer (pH 8.0) in such a manner that a concentration thereof became 14 mg/ml. 0.6 mg of Sulfo-GMBS (available from Dojin Chemical Co.) were added to 1 mL of the fine particle dispersion and were reacted at the room temperature for 1 hour, and the reaction product was purified by a column (trade name: PD-10, manufactured by Pharmacia Bioscience Inc.) to obtain a dispersion of fluorescent fine particles having maleimide groups on the surface.

Then, 3.9 mg of a Fab' fraction, purified from 7.2 mg of anti-theophylline antibody (Cosmo Bio Co.) by a pepsin treatment and a reduction with mercaptoethylamine, were dissolved in a 0.1M HEPES buffer (pH 7.4) in such a manner that a concentration thereof became 1 mg/ml. It was mixed with an equal amount of a solution (1 mg/ml in 0.1M HEPES (pH 7.4)) of fluorescent fine particles having maleimide groups on the surface, then the mixture was agitated overnight at 4°C and purified by gel filtration to obtain antibody-combined fluorescent fine particles-3.

### <Synthesis of fluorescent fine particle dispersion-4>

A dispersion liquid A of fine particles having amino groups on the surface was synthesized in the same manner as in Example 1. Then 5.6 mg of 4,4,4-trifluoro-1-(2-thienyl)-1,3-butanedione, 3.2 mg of terbium (III) chloride hexahydrate and 4.5 mg of the ligand having a triazine ring of exemplary compound 29 were dissolved in 0.5 g of methanol. Then the solution was added to 10 g of the dispersion liquid A of the above-synthesized fine particles having amino groups on the surface, and agitated at the room temperature for 3 hours. Thereafter, methanol in the dispersion liquid was distilled off under a reduced pressure, utilizing an aspirator. After the undissolved substance was filtered off, the dispersion was let to stand at 45°C for 3 days, and then cooled to the room temperature to obtain a fluorescent fine particle dispersion liquid-4. The fluorescent fine particle dispersion liquid-4 showed a strong fluorescence at 543 nm by an irradiation with an excitation light of 480 nm.

### <Synthesis of antibody-combined fine particle-4>

The thus-synthesized fluorescent fine particle dispersion-4 was dispersed in a 0.1 M HEPES buffer (pH 8.0) in such a manner that a concentration thereof became 14 mg/ml. 0.6 mg of Sulfo-GMBS (available from Dojin Chemical Co.) were added to 1 mL of the fine particle dispersion and were reacted at the room temperature for 1 hour, and the reaction product was purified by a column (trade name: PD-10, manufactured by Pharmacia Bioscience Inc.) to obtain a dispersion of fluorescent fine particles having maleimide groups on the surface.

Then, 4.0 mg of a Fab' fraction, purified from 10 mg of anti-folic acid antibody (Cosmo Bio Co.) by a pepsin treatment and a reduction with mercaptoethylamine, were dissolved in a 0.1M HEPES buffer (pH 7.4) in such a manner that a concentration thereof became 1 mg/ml. It was mixed with an equal amount of a solution (1 mg/ml in 0.1M HEPES (pH 7.4)) of fluorescent fine particles having maleimide groups on the surface, then the mixture was agitated overnight at 4°C and purified by gel filtration to obtain antibody-combined fluorescent fine particles-4.

### <Detection>

The antibody-combined fluorescent fine particles 3 and 4 synthesized above were diluted to a fine particle concentration of 0.001 mass % and were mixed in equal amounts. 1 µl of the mixed fine particles was spotted on each of the quartz substrate bonded with theophylline-bonded poly-L-lysine and the quartz substrate bonded with folic acid-bonded poly-L-lysine prepared in the same manner as in Example 1, each of which was then let to stand at the room temperature for 2 hours, and rinsed with purified water. After the water drops on the surface were removed, the quartz substrate was irradiated with lights of 410 and 480 nm and subjected to a fluorescence measurement in a time-resolved mode. As a result, in the quartz substrate bonded with theophylline-bonded poly-L-lysine, a strong fluorescence of a wavelength of 615 nm was observed by the irradiation with the light of 410 nm, but no fluorescence was observed in the irradiation with the light of 480 nm. In the quartz substrate bonded with folic acid-bonded poly-L-lysine, scarce fluorescence was observed by the irradiation with the light of 410 nm, but a strong fluorescence of a wavelength of 543 nm was observed in the irradiation with the light of 480 nm. These results indicate that theophylline and folic acid can be independently detected, by utilizing a fine particle mixed liquid and by changing the wavelength of the irradiation light and the wavelength of the detection light.

### Example 3

### <Synthesis of fluorescent fine particle dispersion-5>

A dispersion liquid A of fine particles having amino groups on the surface was synthesized in the same manner as in Example 1. Then 5.6 mg of 4,4,4-trifluoro-1-(2-thienyl)-1,3-butanedione, 3.2 mg of terbium (III) chloride hexahydrate and 4.5 mg of the ligand having a triazine ring of exemplary compound 5 were dissolved in 0.5 g of methanol. Then the solution was added to 10 g of the dispersion liquid A of the above-synthesized fine particles having amino groups on the surface, and agitated at the room temperature for 3 hours. Thereafter, methanol in the dispersion liquid was distilled off under a reduced pressure, utilizing an aspirator. After the undissolved substance was filtered off, the dispersion was let to stand at 45°C for 3 days, and then cooled to the room temperature to obtain a fluorescent fine particle dispersion liquid-5. The fluorescent fine particle dispersion liquid-5 showed a strong fluorescence at 543 nm by an irradiation with an excitation light of 410 nm.

### <Synthesis of antibody-combined fine particle-5>

The thus-synthesized fluorescent fine particle dispersion-5 was dispersed in a 0.1M HEPES buffer (pH 8.0) in such a manner that a concentration thereof became 14 mg/ml. 0.6 mg of Sulfo-GMBS (available from Dojin Chemical Co.) were added to 1 mL of the fine particle dispersion and were reacted at the room temperature for 1 hour, and the reaction product was purified by a column (trade name: PD-10, manufactured by Pharmacia Bioscience Inc.) to obtain a dispersion of fluorescent fine particles having maleimide groups on the surface.

Then, 4.0 mg of a Fab' fraction, purified from 10 mg of anti-folic acid antibody (Cosmo Bio Co.) by a pepsin treatment and a reduction with mercaptoethylamine, were dissolved in a 0.1M HEPES buffer (pH 7.4) in such a manner that a concentration thereof became 1 mg/ml. It was mixed with an equal amount of a solution (1 mg/ml in 0.1M HEPES (pH 7.4)) of fluorescent fine particles having maleimide groups on the surface, then the mixture was agitated overnight at 4°C and purified by gel filtration to obtain antibody-combined fluorescent fine particles-5.

### <Detection>

The antibody-combined fluorescent fine particles-3 synthesized in Example 2 and the antibody-combined fluorescent fine particles-5 synthesized above were diluted to a fine particle concentration of 0.001 % and were mixed in equal amounts. 1 µl of the mixed fine particles was spotted on each of the quartz substrate bonded with theophylline-bondcd poly-L-lysine and the quartz substrate bonded with folie acid-bonded poly-L-lysine, prepared in the same manner as in Example 1, each of which was then let to stand at the room temperature for 2 hours, and rinsed with purified water. After the water drops on the surface were removed, the quartz substrate was irradiated with a light of 410 nm and subjected to a fluorescence measurement in a time-resolved mode. As a result, in the quartz substrate bonded with theophylline-bonded poly-L-lysine, a strong fluorescence of a wavelength of 615 nm was observed, but a fluorescence of a wavelength of 543 nm was not observed. In the quartz substrate bonded with folic acid-bonded poly-L-lysine, a fluorescence of a wavelength of 615 nm was not observed, but a strong fluorescence of a wavelength of 543 nm was observed. These results indicate that theophylline and folic acid can be independently detected, by utilizing a fine particle mixed liquid and by changing the wavelength of the detection light for the excitation light of a same wavelength.

Now, certain exemplary embodiments of the present invention will be shown below.
[1] A fluorescent polymer fine particle set including at least:
   a first fluorescent polymer fine particle including a polymer fine particle having a core-shell configuration formed by a hydrophobic core and a hydrophilic shell, and a first fluorescent lanthanoid dye incorporated in the polymer fine particle and containing a lanthanoid cation; and
   a second fluorescent polymer fine particle including the polymer fine particle, and a second fluorescent lanthanoid dye different from the first fluorescent lanthanoid dye.
[2] The fluorescent polymer fine particle set described in [1], characterized in that a ligand contained in the first fluorescent lanthanoid dye and a ligand contained in the second fluorescent lanthanoid dye are different from each other.
[3] The fluorescent polymer fine particle set described in [1], characterized in that a difference between an excitation wavelength of the first fluorescent lanthanoid dye and an excitation wavelength of the second fluorescent lanthanoid dye is 10 nm or larger.
[4] The fluorescent polymer fine particle set described in [1], characterized in that a lanthanoid cation contained in the first fluorescent lanthanoid dye and a lanthanoid cation contained in the second fluorescent lanthanoid dye are different from each other.
[5] The fluorescent polymer fine particle set described in any one of [1] to [4], characterized in that a difference between a peak light emission wavelength of the first fluorescent lanthanoid dye and a peak light emission wavelength of the second fluorescent lanthanoid dye is 10 nm or larger.
[6] The fluorescent polymer fine particle set described in [1], characterized in that the polymer fine particle is formed by a hydrophilic shell constituted at least of a hydrophilic (meth)acrylic macromonomer or a hydrophilic vinyl macromonomer, and a hydrophobic core constituted at least of a hydrophobic (meth)acrylic monomer or a hydrophobic vinyl monomer.
[7] The fluorescent polymer fine particle set described in [6], characterized in that the hydrophilic (meth)acrylic macromonomer or the hydrophilic vinyl macromonomer includes a reactive functional group.
[8] The fluorescent polymer fine particle set described in [6], characterized in that the hydrophilic (meth)acrylic macromonomer is represented by a following formula (I) and the hydrophilic vinyl macromonomer is represented by a following formula (II): 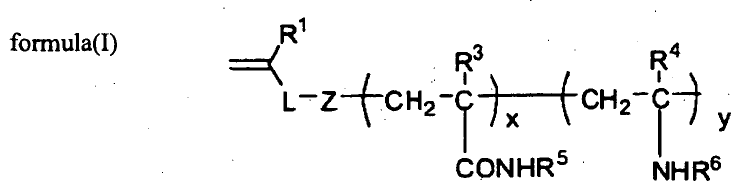 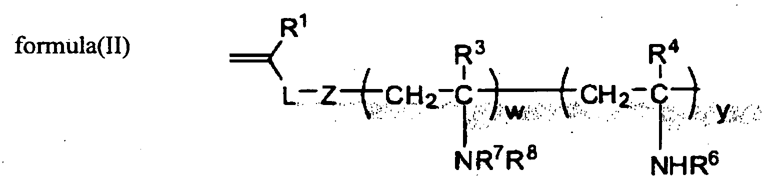 wherein, R¹, R³ and R⁴ each independently represent a hydrogen atom, an alkyl group having 1 to 6 carbon atoms, or a halogen atom; R⁵ represents a hydrogen atom or an alkyl group having 1 to 4 carbon atoms; R⁶ represents a hydrogen atom, a formyl group or an acetyl group; R⁷ and R⁸ each independently represent a hydrogen atom or an alkyl group having 1 to 6 carbon atoms, and also may be bonded with each other to form a nitrogen-containing ring; L represents a divalent linking group; Z represents an atomic group linking to a terminal end of the polymer main chain; x and y each represent a number of repeat equal to or larger than 1; and w represents a number of repeat equal to or larger than 0.
[9] The fluorescent polymer fine particle set described in [1], characterized in that the fluorescent lanthanoid dye includes at least a nitrogen-containing heterocyclic ligand represented by a following formula (L-I): 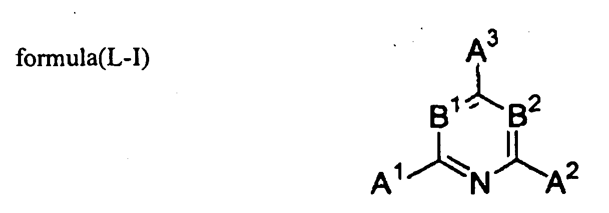 wherein A¹, A² and A³ each represent an atomic group represented by any one of following formulae (L-II) to (L-V), a hydroxyl group, an alkoxy group, an aryloxy group, an alkylamino group, a dialkylamino group, an arylamino group or a diarylamino group, and may be same one another; B¹ and B² each independently represent a nitrogen atom or =C(-R²⁰)-, wherein R²⁰ represents a hydrogen atom or a substituent: 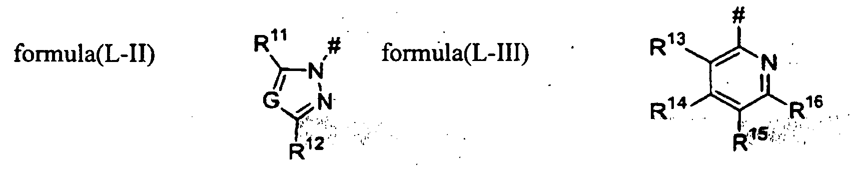 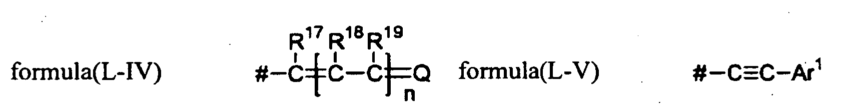 wherein, R¹¹ to R¹⁹ each independently represent a hydrogen atom or a substituent, provided that R¹⁷ and R¹⁸, R¹⁸ and R¹⁹ or R¹⁷ and R¹⁹ may be bonded with each other to form a ring when possible; n represents 0, 1 or 2; G represents a carbon atom or a nitrogen atom that may have a substituent; Q represents an atomic group required for forming a 5- or 6-membered nitrogen-containing heterocycle, which may constitute condensed rings; Ar¹ represents an aromatic carbon ring or an aromatic heterocycle; and # in formulae (L-II) to (L-V) indicates a position to be bonded with the nitrogen-containing heterocycle represented by the formula (L-I).
[10] The fluorescent polymer fine particle set described in [1], characterized in that the lanthanoid cation contained in the fluorescent lanthanoid dye is a trivalent cation.
[11] The fluorescent polymer fine particle set described in [1], characterized in that the lanthanoid cation contained in the fluorescent lanthanoid dye is selected from Nd³⁺, Sm³⁺, Eu³⁺, Tb³⁺, Dy³⁺ and Tm³⁺.
[12] The fluorescent polymer fine particle set described in [1],
   characterized in that the lanthanoid cation contained in the fluorescent lanthanoid dye is selected from Eu³⁺ and Tb³⁺.
[13] A fluorescence detecting complex member set including:
   a first fluorescence detecting complex member, constituted of a first fluorescent polymer fine particle contained in the fluorescent polymer fine particle set described in [1] and a first binding material capable of binding the fluorescent polymer fine particle and a target substance of detection; and
   a second fluorescence detecting complex member, constituted of a second fluorescent polymer fine particle contained in the fluorescent polymer fine particle set and a second binding material different from the first binding material.
[14] The fluorescence detecting complex member set described in [13], characterized in that a target substance of detection of the first binding material and a target substance of detection of the second binding material are different from each other.
[15] A fluorescent polymer fine particle composition including at least:
   a first fluorescent polymer fine particle including a polymer fine particle having a core-shell configuration formed by a hydrophobic core and a hydrophilic shell, and a first fluorescent lanthanoid dye incorporated in the polymer fine particle and containing a lanthanoid cation; and
   a second fluorescent polymer fine particle including the polymer fine particle, and a second fluorescent lanthanoid dye different from the first fluorescent lanthanoid dye.
[16] The fluorescent polymer fine particle composition described in [15], characterized in that the first fluorescent polymer fine particle constitutes, together with a first binding material capable of binding the fluorescent polymer fine particle and a target substance of detection, a first fluorescence detecting complex member; and
   the second fluorescent polymer fine particle constitutes, together with a second binding material different from the first binding material, a second fluorescence detecting complex member.
[17] A fluorescence detecting method including at least:
   forming a first fluorescence detecting complex member from a first fluorescent polymer fine particle contained in the fluorescent polymer fine particle set described in [1] and a first binding material capable of binding the fluorescent polymer fine particle and a target substance of detection;
   forming a second fluorescence detecting complex member from a second fluorescent polymer fine particle contained in the fluorescent polymer fine particle set and a second binding material different from the first binding material;
   bringing the first and second fluorescence detecting complex members into contact with a specimen containing a target substance of detection;
   executing a first detection of detecting the first fluorescent lanthanoid dye contained in the first fluorescence detecting complex member; and
   executing a second detection of detecting the second fluorescent lanthanoid dye contained in the second fluorescence detecting complex member.
[18] A fluorescence detecting method including at least:
   bringing the fluorescence detecting complex members contained in the fluorescence detecting complex member set described in [13] into contact with a specimen containing a target substance of detection;
   executing a first detection of detecting the first fluorescent lanthanoid dye contained in the first fluorescence detecting complex member; and
   executing a second detection of detecting the second fluorescent lanthanoid dye contained in the second fluorescence detecting complex member.
[19] A fluorescence detecting method including at least:
   bringing the fluorescence detecting complex members contained in the fluorescence detecting complex member set described in [13] into contact or a fluorescent polymer fine particle composition described in [16] into contact with a specimen containing a target substance of detection;
   executing a first detection of detecting the first fluorescent lanthanoid dye contained in the first fluorescence detecting complex member; and
   executing a second detection of detecting the second fluorescent lanthanoid dye contained in the second fluorescence detecting complex member.

## Claims

1. A fluorescent polymer fine particle set comprising at least:
a first fluorescent polymer fine particle including a polymer fine particle having a core-shell configuration formed by a hydrophobic core and a hydrophilic shell, and a first fluorescent lanthanoid dye incorporated in the polymer fine particle and containing a lanthanoid cation; and
a second fluorescent polymer fine particle including the polymer fine particle, and a second fluorescent lanthanoid dye different from the first fluorescent lanthanoid dye.

2. The fluorescent polymer fine particle set according to claim 1, **characterized in that** a ligand contained in the first fluorescent lanthanoid dye and a ligand contained in the second fluorescent lanthanoid dye are different from each other.

3. The fluorescent polymer fine particle, set according to claim 1 or 2, **characterized in that** a difference between an excitation wavelength of the first fluorescent lanthanoid dye and an excitation wavelength of the second fluorescent lanthanoid dye is 10 nm or larger.

4. The fluorescent polymer fine particle set according to any one of claims 1 to 3, **characterized in that** a lanthanoid cation contained in the first fluorescent lanthanoid dye and a lanthanoid cation contained in the second fluorescent lanthanoid dye are different from each other.

5. The fluorescent polymer fine particle set according to any one of claims 1 to 4, **characterized in that** a difference between a peak light emission wavelength of the first fluorescent lanthanoid dye and a peak light emission wavelength of the second fluorescent lanthanoid dye is 10 nm or larger.

6. The fluorescent polymer fine particle set according to any one of claims 1 to 5, **characterized in that** the polymer fine particle is formed by a hydrophilic shell constituted at least of a hydrophilic (meth)acrylic macromonomer or a hydrophilic vinyl macromonomer, and a hydrophobic core constituted at least of a hydrophobic (meth)acrylic monomer or a hydrophobic vinyl monomer.

7. The fluorescent polymer fine particle set according to claim 6, **characterized in that** the hydrophilic (meth)acrylic macromonomer or the hydrophilic vinyl macromonomer includes a reactive functional group.

8. The fluorescent polymer fine particle set according to claim 6 or 7, **characterized in that** the hydrophilic (meth)acrylic macromonomer is represented by a following formula (I) and the hydrophilic vinyl macromonomer is represented by a following formula (II): 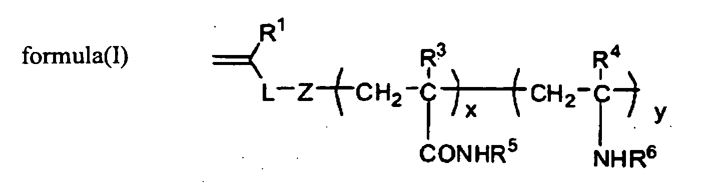 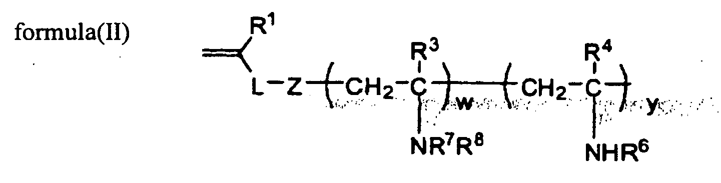 wherein, R¹, R³ and R⁴ each independently represent a hydrogen atom, an alkyl group having 1 to 6 carbon atoms, or a halogen atom; R⁵ represents a hydrogen atom or an alkyl group having 1 to 4 carbon atoms; R⁶ represents a hydrogen atom, a formyl group or an acetyl group; R⁷ and R⁸ each independently represent a hydrogen atom or an alkyl group having 1 to 6 carbon atoms, and also may be bonded with each other to form a nitrogen-containing ring; L represents a divalent linking group; Z represents an atomic group linking to a terminal end of the polymer main chain; x and y each represent a number of repeat equal to or larger than 1; and w represents a number of repeat equal to or larger than 0.

9. The fluorescent polymer fine particle set according to any one of claims 1 to 8, **characterized in that** the fluorescent lanthanoid dye comprises at least a nitrogen-containing heterocyclic ligand represented by a following formula (L-I): 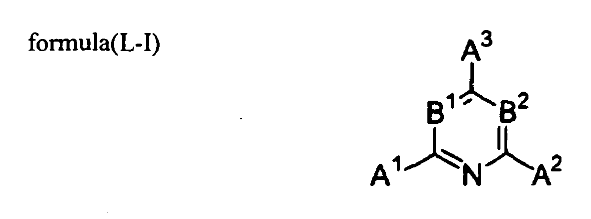 wherein A¹, A² and A³ each represent an atomic group represented by any one of following formulae (L-II) to (L-V), a hydroxyl group, an alkoxy group, an aryloxy group, an alkylamino group, a dialkylamino group, an arylamino group or a diarylamino group, and may be same one another; B¹ and B² each independently represent a nitrogen atom or =C(-R²⁰)-, wherein R²⁰ represents a hydrogen atom or a substituent: 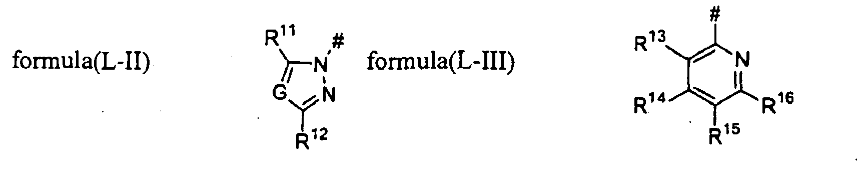 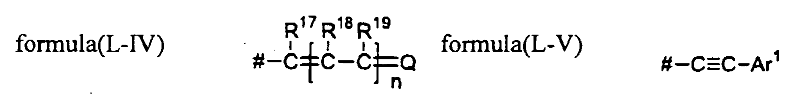 wherein R¹¹ to R¹⁹ each independently represent a hydrogen atom or a substituent, provided that R¹⁷ and R¹⁸, R¹⁸ and R¹⁹ or R¹⁷ and R¹⁹ may be bonded with each other to form a ring when possible; n represents 0, 1 or 2; G represents a carbon atom or a nitrogen atom that may have a substituent; Q represents an atomic group required for forming a 5- or 6-membered nitrogen-containing heterocycle, which may constitute condensed rings; Ar¹ represents an aromatic carbon ring or an aromatic heterocycle; and # in formulas (L-II) to (L-V) indicates a position to be bonded with the nitrogen-containing heterocycle represented by the formula (L-I).

10. A fluorescence detecting complex member set comprising:
a first fluorescence detecting complex member, constituted of a first fluorescent polymer fine particle contained in the fluorescent polymer fine particle set according to any one of claims 1 to 9 and a first binding material capable of binding the fluorescent polymer fine particle and a target substance of detection; and
a second fluorescence detecting complex member, constituted of a second fluorescent polymer fine particle contained in the fluorescent polymer fine particle set according to any one of claims 1 to 9 and a second binding material different from the first binding material.

11. The fluorescence detecting complex member set according to claim 10, wherein a target substance of detection of the first binding material and a target substance of detection of the second binding material are different from each other.

12. Use of a fluorescent polymer fire particle set in a composition for a display apparatus;
the fluorescent polymer fine particle set comprising at least:
a first fluorescent polymer fine particle including a polymer fine particle having a core-shell configuration formed by a hydrophobic core and a hydrophilic shell, and a first fluorescent lanthanoid dye incorporated in the polymer fine particle and containing a lanthanoid cation; and
a second fluorescent polymer fine particle including the polymer fine particle, and a second fluorescent lanthanoid dye different from the first fluorescent lanthanoid dye.

13. Use of the fluorescent polymer fine particle set according to claim 12, wherein the first fluorescent polymer fine particle constitutes, together with a first binding material capable of binding the fluorescent polymer fine particle and a target substance of detection, a first fluorescence detecting complex member; and
the second fluorescent polymer fine particle constitutes, together with a second binding material different from the first binding material, a second fluorescence detecting complex member.

14. A fluorescence detecting method comprising at least:
forming a first fluorescence detecting complex member from a first fluorescent polymer fine particle contained in the fluorescent polymer fine particle set according to any one of claims 1 to 9 and a first binding material capable of binding the fluorescent polymer fine particle and a target substance of detection;
forming a second fluorescence detecting complex member from a second fluorescent polymer fine particle contained in the fluorescent polymer fine particle set according to any one of claims 1 to 9 and a second binding material different from the first binding material;
bringing the first and second fluorescence detecting complex members into contact with a specimen containing a target substance of detection;
executing a first detection of detecting the first fluorescent lanthanoid dye contained in the first fluorescence detecting complex member; and
executing a second detection of detecting the second fluorescent lanthanoid dye contained in the second fluorescence detecting complex member.

15. A fluorescence detecting method comprising at least:
bringing fluorescence detecting complex members contained in the fluorescence detecting complex member set according to claim 10 or 11 into contact with a specimen containing a target substance of detection;
executing a first detection of detecting the first fluorescent lanthanoid dye contained in the first fluorescence detecting complex member; and
executing a second detection of detecting the second fluorescent lanthanoid dye contained in the second fluorescence detecting complex member.

## Patentansprüche

1. Set von fluoreszierenden Polymer-Feinpartikeln, umfassend zumindest:
einen ersten fluoreszierenden Polymer-Feinpartikel, umfassend einen Polymer-Feinpartikel mit einer Kern/Schale-Konfiguration, gebildet aus einem hydrophoben Kern und einer hydrophilen Schale, und einen ersten fluoreszierenden Lanthanoid-Farbstoff, der in den Polymer-Feinpartikel eingearbeitet ist und ein Lanthanoid-Kation enthält; und
einen zweiten fluoreszierenden Polymer-Feinpartikel, umfassend den Polymer-Feinpartikel und einen zweiten fluoreszierenden Lanthanoid-Farbstoff, der sich von dem ersten fluoreszierenden Lanthanoid-Farbstoff unterscheidet.

2. Set von fluoreszierenden Polymer-Feinpartikeln gemäss Anspruch 1, **dadurch gekennzeichnet, dass** ein Ligand, der in dem ersten fluoreszierenden Lanthanoid-Farbstoff enthalten ist, und ein Ligand, der in dem zweiten fluoreszierenden Lanthanoid-Farbstoff enthalten ist, sich voneinander unterscheiden.

3. Set von fluoreszierenden Polymer-Feinpartikeln gemäss Anspruch 1 oder 2, **dadurch gekennzeichnet, dass** der Unterschied zwischen einer Anregungswellenlänge des ersten fluoreszierenden Lanthanoid-Farbstoffs und einer Anregungswellenlänge des zweiten fluoreszierenden Lanthanoid-Farbstoffs 10 nm oder mehr beträgt.

4. Set von fluoreszierenden Polymer-Feinpartikeln gemäss irgendeinem der Ansprüche 1 bis 3, **dadurch gekennzeichnet, dass** ein Lanthanoid-Kation, das in dem ersten fluoreszierenden Lanthanoid-Farbstoff enthalten ist, und ein Lanthanoid-Kation, das in dem zweiten fluoreszierenden Lanthanoid-Farbstoff enthalten ist, sich voneinander unterscheiden.

5. Set von fluoreszierenden Polymer-Feinpartikeln gemäss irgendeinem der Ansprüche 1 bis 4, **dadurch gekennzeichnet, dass** der Unterschied zwischen einer Peak-Lichtemissionswellenlänge des ersten fluoreszierenden Lanthanoid-Farbstoffs und einer Peak-Lichtemissionswellenlänge des zweiten fluoreszierenden Lanthanoid-Farbstoffs 10 nm oder mehr beträgt.

6. Set von fluoreszierenden Polymer-Feinpartikeln gemäss irgendeinem der Ansprüche 1 bis 5, **dadurch gekennzeichnet, dass** der Polymer-Feinpartikel aus einer hydrophilen Schale, die aus zumindest einem hydrophilen (Meth)acryl-Makromonomer oder einem hydrophilen Vinyl-Makromonomer aufgebaut ist, und einem hydrophoben Kern, der aus zumindest einem hydrophoben (Meth)acrylmonomer oder einem hydrophoben Vinylmonomer aufgebaut ist, gebildet wird.

7. Set von fluoreszierenden Polymer-Feinpartikeln gemäss Anspruch 6, **dadurch gekennzeichnet, dass** das hydrophile (Meth)acryl-Makromonomer oder das hydrophile Vinyl-Makromonomer eine reaktive funktionelle Gruppe umfasst.

8. Set von fluoreszierenden Polymer-Feinpartikeln gemäss Anspruch 6 oder 7, **dadurch gekennzeichnet, dass** das hydrophile (Meth)acryl-Makromonomer durch die folgende Formel (I) dargestellt wird und das hydrophile Vinyl-Makromonomer durch die folgende Formel (II) dargestellt wird: 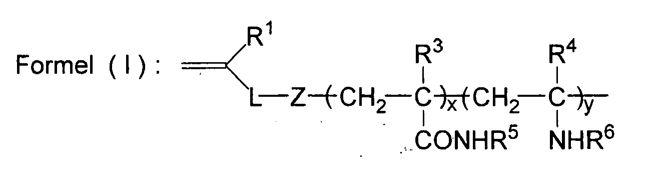 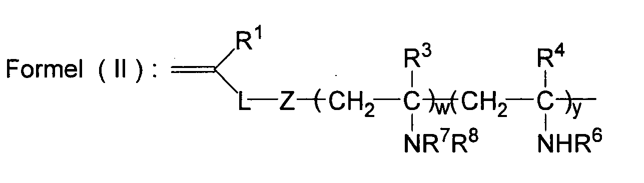 worin R¹, R³ und R⁴ jeweils unabhängig voneinander ein Wasserstoffatom, eine Alkylgruppe mit 1 bis 6 Kohlenstoffatomen oder ein Halogenatom darstellen; R⁵ ein Wasserstoffatom oder eine Alkylgruppe mit 1 bis 4 Kohlenstoffatomen darstellt; R⁶ ein Wasserstoffatom, eine Formylgruppe oder eine Acetylgruppe darstellt; R⁷ und R⁸ jeweils unabhängig voneinander ein Wasserstoffatom oder eine Alkylgruppe mit 1 bis 6 Kohlenstoffatomen darstellen und auch miteinander verbunden sein können, um einen stickstoffhaltigen Ring zu bilden; L eine divalente Verknüpfungsgruppe darstellt; Z eine atomare Gruppe darstellt, die an ein Ende der Polymer-Hauptkette bindet; x und y jeweils eine Wiederholungszahl darstellen, die gleich oder grösser als 1 ist; und w eine Wiederholungszahl darstellt, die gleich oder grösser als 0 ist.

9. Set von fluoreszierenden Polymer-Feinpartikeln gemäss irgendeinem der Ansprüche 1 bis 8, **dadurch gekennzeichnet, dass** der fluoreszierende Lanthanoid-Farbstoff mindestens einen stickstoffhaltigen heterocyclischen Liganden umfasst, der durch die folgende Formel (L-I) dargestellt ist: 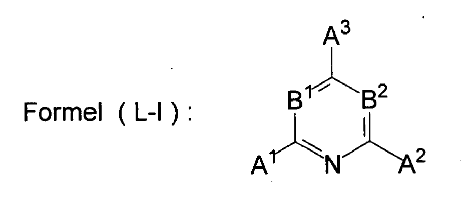 worin A¹, A² und A³ jeweils eine atomare Gruppe, die durch irgendeine der folgenden Formeln (L-II) bis (L-V) dargestellt wird, eine Hydroxylgruppe, eine Alkoxygruppe, eine Aryloxygruppe, eine Alkylaminogruppe, eine Dialkylaminogruppe, eine Arylaminogruppe oder eine Diarylaminogruppe darstellen und zueinander gleich sein können; B¹ und B² jeweils unabhängig voneinander ein Stickstoffatom oder =C(-R²⁰)- darstellen, worin R²⁰ ein Wasserstoffatom oder einen Substituenten darstellt: 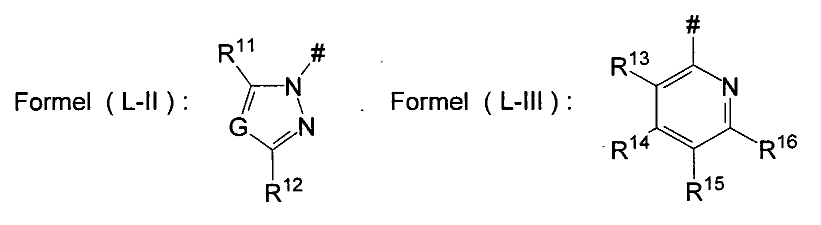 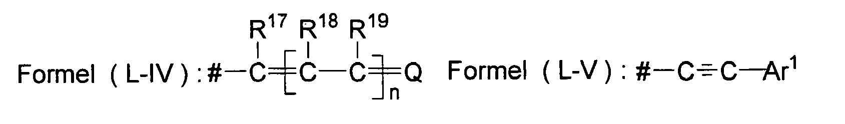 worin R¹¹ bis R¹⁹ jeweils unabhängig voneinander ein Wasserstoffatom oder einen Substituenten darstellen, vorausgesetzt, dass R¹⁷ und R¹⁸, R¹⁸ und R¹⁹ oder R¹⁷ und R¹⁹ miteinander binden können, um einen Ring zu bilden, falls dies möglich ist; n 0, 1 oder 2 darstellt; G ein Kohlenstoffatom oder ein Stickstoffatom darstellt, die einen Substituenten aufweisen können; Q eine atomare Gruppe darstellt, die zum Bilden eines 5- oder 6-gliedrigen stickstoffhaltigen Heterocyclus notwendig ist, der kondensierte Ringe aufbauen darf; Ar¹ einen aromatischen Kohlenstoffring oder einen aromatischen Heterocyclus darstellt; und # in den Formeln (L-II) bis (L-V) eine Position anzeigt, die mit dem stickstoffhaltigen Heterocyclus, der durch die Formel (L-I) dargestellt ist, verbunden ist.

10. Set von Komplexelementen zur Fluoreszenzdetektierung, umfassend:
ein erstes Fluoreszenz-detektierendes Komplexelement, aufgebaut aus einem ersten fluoreszierenden Polymer-Feinpartikel, der in dem Set von fluoreszierenden Polymer-Feinpartikeln gemäss irgendeinem der Ansprüche 1 bis 9 enthalten ist, und einem ersten Bindemittelmaterial, das den fluoreszierenden Polymer-Feinpartikel und die zu detektierende Zielsubstanz binden kann; und
ein zweites Fluoreszenz-detektierendes Komplexelement, aufgebaut aus einem zweiten fluoreszierenden Polymer-Feinpartikel, der in dem Set von fluoreszierenden Polymer-Feinpartikeln gemäss irgendeinem der Ansprüche 1 bis 9 enthalten ist, und einem zweiten Bindemittelmaterial, das sich von dem ersten Bindemittelmaterial unterscheidet.

11. Set von Komplexelementen zur Fluoreszenzdetektierung gemäss Anspruch 10, worin die zu detektierende Zielsubstanz des ersten Bindemittelmaterials und die zu detektierende Zielsubstanz des zweiten Bindemittelmaterials sich voneinander unterscheiden.

12. Verwendung eines Sets von fluoreszierenden Polymer-Feinpartikeln in einer Zusammensetzung für eine Anzeigevorrichtung; wobei das Set von fluoreszierenden Polymer-Feinpartikeln mindestens umfasst:
einen ersten fluoreszierenden Polymer-Feinpartikel, umfassend einen Polymer-Feinpartikel mit einer Kern/Schale-Konfiguration, gebildet aus einem hydrophoben Kern und einer hydrophilen Schale, und einen ersten fluoreszierenden Lanthanoid-Farbstoff, der in den Polymer-Feinpartikel eingearbeitet ist und ein Lanthanoid-Kation enthält; und
einen zweiten fluoreszierenden Polymer-Feinpartikel, umfassend den Polymer-Feinpartikel und einen zweiten fluoreszierenden Lanthanoid-Farbstoff, der sich von dem ersten fluoreszierenden Lanthanoid-Farbstoff unterscheidet.

13. Verwendung des Sets von fluoreszierenden Polymer-Feinpartikeln gemäss Anspruch 12, worin der erste fluoreszierende Polymer-Feinpartikel zusammen mit einem ersten Bindemittelmaterial, das den fluoreszierenden Polymer-Feinpartikel und eine zu detektierende Zielsubstanz binden kann, ein erstes Fluoreszenz-detektierendes Komplexelement bildet; und
der zweite fluoreszierende Polymer-Feinpartikel zusammen mit einem zweiten Bindemittelmaterial, das sich von dem ersten Bindemittelmaterial unterscheidet, ein zweites Fluoreszenz-detektierendes Komplexelement bildet.

14. Verfahren zur Detektierung von Fluoreszenz, mindestens umfassend:
Bilden eines ersten Fluoreszenz-detektierenden Komplexelements aus einem ersten fluoreszierenden Polymer-Feinpartikel, der in dem Set von fluoreszierenden Polymer-Feinpartikeln gemäss irgendeinem der Ansprüche 1 bis 9 enthalten ist, und einem ersten Bindemittelmaterial, das den fluoreszierenden Polymer-Feinpartikel und die zu detektierende Zielsubstanz binden kann;
Bilden eines zweiten Fluoreszenz-detektierenden Komplexelements aus einem zweiten fluoreszierenden Polymer-Feinpartikel, der in dem Set von fluoreszierenden Polymer-Feinpartikeln gemäss irgendeinem der Ansprüche 1 bis 9 enthalten ist, und einem zweiten Bindemittelmaterial, das sich von dem ersten Bindemittelmaterial unterscheidet;
In-Kontakt-Bringen des ersten und des zweiten Fluoreszenz-detektierenden Komplexelements mit einer Probe, die die zu detektierende Zielsubstanz enthält;
Durchführen einer ersten Detektierung zum Detektieren des ersten fluoreszierenden Lanthanoid-Farbstoffs, der in dem ersten Fluoreszenz-detektierenden Komplexelement enthalten ist; und
Durchführen einer zweiten Detektierung zum Detektieren des zweiten fluoreszierenden Lanthanoid-Farbstoffs, der in dem zweiten Fluoreszenz-detektierenden Komplexelement enthalten ist.

15. Verfahren zum Detektieren von Fluoreszenz, mindestens umfassend:
In-Kontakt-Bringen der Fluoreszenz-detektierenden Komplexelemente, die in dem Set von Komplexelementen zur Detektierung von Fluoreszenz gemäss Anspruch 10 oder 11 enthalten sind, mit einer Probe, die die zu detektierende Zielsubstanz enthält;
Durchführen einer ersten Detektierung zum Detektieren des ersten fluoreszierenden Lanthanoid-Farbstoffs, der in dem ersten Fluoreszenz-detektierenden Komplexelement enthalten ist; und
Durchführen einer zweiten Detektierung zum Detektieren des zweiten fluoreszierenden Lanthanoid-Farbstoffs, der in dem zweiten Fluoreszenz-detektierenden Komplexelement enthalten ist.

## Revendications

1. Ensemble de particules fines de polymère fluorescent comprenant au moins :
une première particule fine de polymère fluorescent incluant une particule fine de polymère ayant une configuration coeur-coquille formée par un coeur hydrophobe et une coquille hydrophile, et un premier colorant lanthanoïde fluorescent incorporé dans la particule fine de polymère et contenant un cation lanthanoïde ; et
une seconde particule fine de polymère fluorescent comportant la particule fine de polymère, et un second colorant lanthanoïde fluorescent différent du premier colorant lanthanoïde fluorescent.

2. Ensemble de particules fines de polymère fluorescent selon la revendication 1, **caractérisé en ce qu'**un ligand contenu dans le premier colorant lanthanoïde fluorescent et un ligand contenu dans le second colorant lanthanoïde fluorescent sont différents l'un de l'autre.

3. Ensemble de particules fines de polymère fluorescent selon la revendication 1 ou 2, **caractérisé en ce qu'**une différence entre une longueur d'onde d'excitation du premier colorant lanthanoïde fluorescent et une longueur d'onde d'excitation du second colorant lanthanoïde fluorescent est de 10 nm ou davantage.

4. Ensemble de particules fines de polymère fluorescent selon l'une quelconque des revendications 1 à 3, **caractérisé en ce qu'**un cation lanthanoïde contenu dans le premier colorant lanthanoïde fluorescent et un cation lanthanoïde contenu dans le second colorant lanthanoïde fluorescent sont différents l'un de l'autre.

5. Ensemble de particules fines de polymère fluorescent selon l'une quelconque des revendications 1 à 4, **caractérisé en ce qu'**une différence entre une longueur d'onde maximale d'émission de lumière du premier colorant lanthanoïde fluorescent et une longueur d'onde maximale d'émission de lumière du second colorant lanthanoïde fluorescent est de 10 nm ou davantage.

6. Ensemble de particules fines de polymère fluorescent selon l'une quelconque des revendications 1 à 5, **caractérisé en ce que** la particule fine de polymère est formée par une coquille hydrophile constituée d'au moins un macromonomère (méth)acrylique hydrophile ou un macromonomère vinylique hydrophile, et d'un coeur hydrophobe constitué d'au moins un monomère (méth)acrylique hydrophobe ou un monomère vinylique hydrophobe.

7. Ensemble de particules fines de polymère fluorescent selon la revendication 6, **caractérisé en ce que** le macromonomère (méth)acrylique hydrophile ou le macromonomère vinylique hydrophile inclut un groupe fonctionnel réactif.

8. Ensemble de particules fines de polymère fluorescent selon la revendication 6 ou 7, **caractérisé en ce que** le macromonomère (méth)acrylique hydrophile est représenté par la formule (I) suivante et le macromonomère vinylique hydrophile est représenté par la formule (II) suivante : 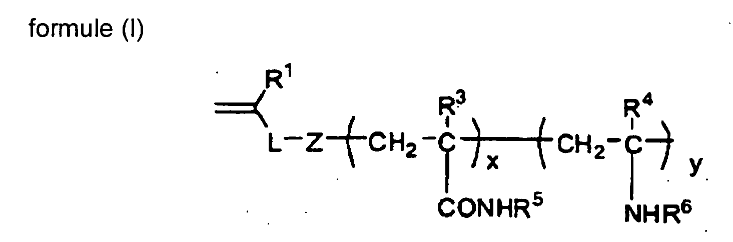 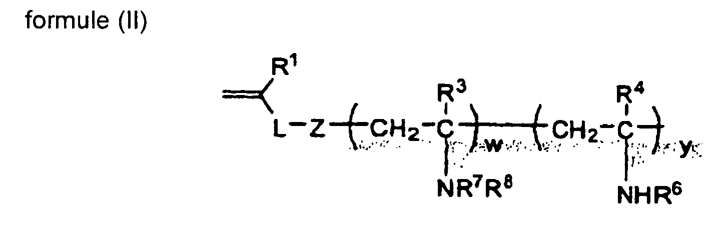 dans lesquelles, R¹, R³ et R⁴ représentent chacun indépendamment un atome d'hydrogène, un groupe alkyle ayant 1 à 6 atomes de carbone, ou un atome d'halogène ; R⁵ représente un atome d'hydrogène ou un groupe alkyle ayant 1 à 4 atomes de carbone ; R⁶ représente un atome d'hydrogène, un groupe formyle ou un groupe acétyle ; R⁷ et R⁸ représentent chacun indépendamment un atome d'hydrogène ou un groupe alkyle ayant 1 à 6 atomes de carbone, et peuvent également être liés l'un à l'autre pour former un cycle contenant de l'azote ; L représente un groupe de liaison divalent ; Z représente un groupe atomique se liant à une extrémité terminale de la chaîne principale de polymère ; x et y représentent chacun un nombre de répétitions égal ou supérieur à 1 ; et w représente un nombre de répétitions égal ou supérieur à 0.

9. Ensemble de particules fines de polymère fluorescent selon l'une quelconque des revendications 1 à 8, **caractérisé en ce que** le colorant lanthanoïde fluorescent comprend au moins un ligand hétérocyclique contenant de l'azote représenté par la formule (L-I) suivante : 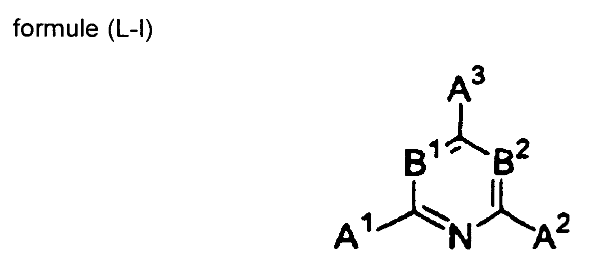 dans laquelle A¹, A² et A³ représentent chacun un groupe atomique représenté par l'une quelconque des formules (L-II) à (L-V) suivantes, un groupe hydroxyle, un groupe alcoxy, un groupe aryloxy, un groupe alkylamino, un groupe dialkylamino, un groupe arylamino ou un groupe diarylamino, et les uns peuvent être les mêmes que les autres ; B¹ et B² représentent chacun indépendamment un atome d'azote ou =C(-R²⁰)-, dans lequel R²⁰ représente un atome d'hydrogène ou un substituant : 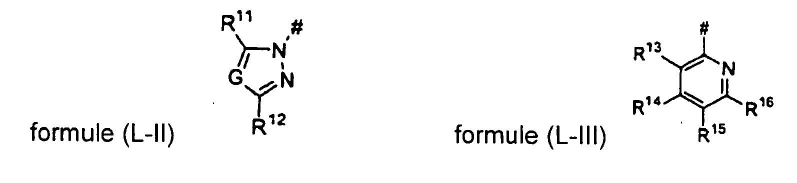 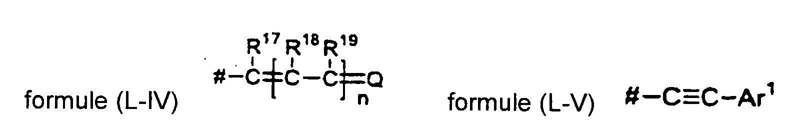 dans lesquelles R¹¹ à R¹⁹ représentent chacun indépendamment un atome d'hydrogène ou un substituant, à la condition que R¹⁷ et R¹⁸, R¹⁸ et R¹⁹ ou R¹⁷ et R¹⁹ puissent être liés l'un à l'autre pour former un cycle lorsque c'est possible ; n représente 0, 1 ou 2 ; G représente un atome de carbone ou un atome d'azote qui peuvent avoir un substituant ; Q représente un groupe atomique requis pour former un hétérocycle contenant de l'azote de 5 ou 6 membres, qui peuvent constituer des cycles condensés ; Ar¹ représente un cycle carboné aromatique ou un hétérocycle aromatique ; et # dans les formules (L-II) à (L-V) indique une position devant être liée à l'hétérocycle contenant de l'azote représenté par la formule (L-I).

10. Ensemble de membres complexes de détection de fluorescence comprenant :
un premier membre complexe de détection de fluorescence, constitué d'une première particule fine de polymère fluorescent contenue dans l'ensemble de particules fines de polymère fluorescent selon l'une quelconque des revendications 1 à 9 et d'un premier matériau de liaison capable de lier la particule fine de polymère fluorescent et une substance cible de détection ; et
un second membre complexe de détection de fluorescence, constitué d'une seconde particule fine de polymère fluorescent contenue dans l'ensemble de particules fines de polymère fluorescent selon l'une quelconque des revendications 1 à 9 et d'un second matériau de liaison différent du premier matériau de liaison.

11. Ensemble de membres complexes de détection de fluorescence selon la revendication 10, dans lequel une substance cible de détection du premier matériau de liaison et une substance cible de détection du second matériau de liaison sont différentes l'une de l'autre.

12. Utilisation d'un ensemble de particules fines de polymère fluorescent dans une composition pour un appareil d'affichage ;
l'ensemble de particules fines de polymère fluorescent comprenant au moins :
une première particule fine de polymère fluorescent incluant une particule fine de polymère ayant une configuration coeur-coquille formée par un coeur hydrophobe et une coquille hydrophile, et un premier colorant lanthanoïde fluorescent incorporé dans la particule fine de polymère et contenant un cation lanthanoïde ; et
une seconde particule fine de polymère fluorescent incluant la particule fine de polymère, et un second colorant lanthanoïde fluorescent différent du premier colorant lanthanoïde fluorescent.

13. Utilisation de l'ensemble de particules fines de polymère fluorescent selon la revendication 12, dans laquelle la première particule fine de polymère fluorescent constitue, conjointement avec un premier matériau de liaison capable de lier la particule fine de polymère fluorescent et une substance cible de détection, un premier membre complexe de détection de fluorescence ; et
la seconde particule fine de polymère fluorescent constitue, conjointement avec un second matériau de liaison différent du premier matériau de liaison, un second membre complexe de détection de fluorescence.

14. Procédé de détection de fluorescence comprenant au moins :
la formation d'un premier membre complexe de détection de fluorescence à partir d'une première particule fine de polymère fluorescent contenue dans l'ensemble de particules fines de polymère fluorescent selon l'une quelconque des revendications 1 à 9 et d'un premier matériau de liaison capable de lier la particule fine de polymère fluorescent et une substance cible de détection ;
la formation d'un second membre complexe de détection de fluorescence à partir d'une seconde particule fine de polymère fluorescent contenue dans l'ensemble de particules fines de polymère fluorescent selon l'une quelconque des revendications 1 à 9 et d'un second matériau de liaison différent du premier matériau de liaison ;
la mise en contact du premier et du second membres complexes de détection de fluorescence avec un échantillon contenant une substance cible de détection ;
la réalisation d'une première détection consistant à détecter le premier colorant lanthanoïde fluorescent contenu dans le premier membre complexe de détection de fluorescence ; et
la réalisation d'une seconde détection consistant à détecter le second colorant lanthanoïde fluorescent contenu dans le second membre complexe de détection de fluorescence.

15. Procédé de détection de fluorescence comprenant au moins :
la mise en contact de membres complexes de détection de fluorescence contenus dans l'ensemble de membres complexes de détection de fluorescence selon la revendication 10 ou 11 avec un échantillon contenant une substance cible de détection ;
la réalisation d'une première détection consistant à détecter le premier colorant lanthanoïde fluorescent contenu dans le premier membre complexe de détection de fluorescence ; et
la réalisation d'une seconde détection consistant à détecter le second colorant lanthanoïde fluorescent contenu dans le second membre complexe de détection de fluorescence.
